# FASCICULE DE BREVET EUROPEEN

(11) **EP 0 868 512 B1**
(45) Date de publication et mention de la délivrance du brevet: **26.10.2005**
(21) Numéro de dépôt: 96943177.4
(22) Date de dépôt: 20.12.1996
(51) Int. Cl.: C12N 15/12, C12N 15/86, C12N 5/10, C07K 14/47, C07K 14/82, A61K 39/395, A61K 48/00, C12Q 1/68, G01N 33/574, G01N 33/68

(54) **SEQUENCES NUCLEOTIDIQUES, PROTEINES, MEDICAMENTS ET AGENTS DIAGNOSTIQUES UTILES DANS LE TRAITEMENT DU CANCER**
NUKLEOTIDSEQUENZEN, PROTEINE, MEDIKAMENTE UND DIAGNOSEAGENTIEN ZUR ANWENDUNG IN KREBSBEHANDLUNG
NUCLEOTIDE SEQUENCES, PROTEINS, DRUGS AND DIAGNOSTIC AGENTS FOR TREATING CANCER

(30) Priorité: 20.12.1995 FR 9515146; 18.04.1996 FR 9604853
(43) Date de publication de la demande: 07.10.1998
(73) Titulaire: Cerenis, 75011 Paris (FR)
(72) Inventeur: TELERMAN, Adam, F-75007 Paris (FR); AMSON, Robert, F-75005 Paris (FR); COHEN, Daniel, F-94120 Fontenay-sous-Bois (FR)
(74) Mandataire: Warcoin, Jacques
(86) Numéro de dépôt international: PCT/FR1996/002061
(87) Numéro de publication internationale: WO 1997/022695

(56) Documents cités:
- WO-A-95/11301
- WO-A-95/19367
- J. BIOL. CHEM., vol. 268, no. 28, 5 Octobre 1993, pages 21318-21327, XP002013539 LEE: "Purification, molecular cloning and sequencing of phospholipase C-beta4"
- HUMAN MOLECULAR GENETICS, vol. 3, no. 3, 1994, pages 465-470, XP002013540 TODA: "Isolation and characterization of a novel gene encoding nuclear protein at a locus (D11S636) tightly linked to multiple endocrine neoplasia type 1 (MEN1)"
- DEVELOPMENT, vol. 117, no. 4, 1993, pages 1333-1343, XP000601972 DELLA: "Isolation and characterization of murine homologues of the Drosophila seven in absentia gene (sina)" & DATABASE EMBL ID: MMSIAH1A, AC=Z19579,
- FEBS LETT., vol. 374, no. 3, 6 Novembre 1995, pages 384-386, XP002013541 GUENAL: "Studies of specific gene induction during apoptosis of cell lines conditionally immortalized by SV40"
- ONCOGENE, vol. 9, no. 12, 1994, pages 3743-3751, XP000602314 ZHAN: "Induction of bax by genotoxic stress in human cells correlates with normal p53 status and apoptosis"
- SCIENCE, vol. 257, 14 Août 1992, pages 967-971, XP000508268 LIANG: "Differential display of eukaryotic messenger RMA by means of the polymerase chain reaction" cité dans la demande
- NUCLEIC ACIDS REASEARCH, vol. 19, no. 14, 25 Juillet 1991, page 4008 XP002013542 DON: ""Touchdown" PCR to circumvent spurious priming during gene amplification" cité dans la demande
- DATABASE EMBL ID: HS152227, AC= H72152, 2 Novembre 1995 XP002019920 & UNPUBLISHED, 1995, HILLIER ET AL.:
- DATABASE EMBL ID: HS49264, AC=N31049, 12 Janvier 1996 XP002019921 & UNPUBLISHED, 1996, HILLIER ET AL.:
- PROC. NATL ACAD. SCI., vol. 93, no. 9, 30 Avril 1996, pages 3953-3957, XP002032914 AMSON ET AL.: "Isolation of 10 differentially expressed cDNAs in p53-induced apoptosis : activation of the vertebrate homologue of Drosophila seven in absentia gene"
- PROC. NATL ACAD. SCI., vol. 93, no. 17, 20 Août 1996, pages 9039-9042, XP000611649 NEMANI ET AL.: "Activation of the human homologue of the Drosophila sina gene in apoptosis and tumor suppression"
- NATURE, vol. 375, 29 Juin 1995, pages 754-760, XP002032915 SHERRINGTON ET AL.: "Cloning of a gene bearing missense mutations in early-onset familial Alzheimer's disease" cité dans la demande
- AUSTRALIAN AND NEW ZEALAND JOURNAL OF MEDICINE, vol. 25, no. 6, Décembre 1995, pages 845-851, XP000610669 DELLA N G ET AL: "A COMBINED GENETIC AND BIOCHEMICAL APPROACH TO MAMMALIAN SIGNAL TRANSDUCTION"

## Description

La présente invention concerne la mise en évidence de gènes impliqués dans les voies moléculaires de la suppression tumorale et l'utilisation des gènes ainsi mis en évidence pour le traitement de certains dysfonctionnements géniques, notamment les cancers.

La présente invention a été rendue possible par l'isolement d'ADNc correspondant à des ARN messagers exprimés ou réprimés lors du processus d'apoptose induit par le gène suppresseur p53.

Une analyse globale des événements moléculaires intervenant au cours du cycle cellulaire lors du développement et de l'apoptose cellulaire est nécessaire pour mieux comprendre l'importance du gène p53 dans le processus de suppression tumorale ou, au contraire, de cancérisation.

La transformation d'une cellule normale en cellule tumorale est un processus qui se déroule en plusieurs étapes et qui nécessite une suite d'événements moléculaires. Au niveau physiologique, ces événements se traduiront par une indépendance de la cellule tumorale vis-à-vis des signaux extérieurs ainsi que par une dérégulation interne menant à une croissance incontrôlée.

Deux groupes de gènes sont responsables de cette transformation dite "maligne", d'une part, les oncogènes, d'autre part, les gènes suppresseurs ou anti-oncogènes. Les oncogènes, en raison de leur dérégulation dans le cancer (résultant le plus souvent d'une mutation ou d'une translocalisation) induiront un signal positif qui favorisera la croissance néoplasique. Au contraire, les gènes suppresseurs, du fait de leur délétion, de l'absence de leur expression par mutation du promoteur, par exemple, ou encore de mutations qui modifieront la structure et la fonction de la protéine, seront incapables dans le cancer de fournir le signal qui lui, normalement, devrait freiner cette croissance anormale. En conséquence, le dysfonctionnement des gènes suppresseurs contribue à la transformation néoplasique.

L'objet de la présente invention est l'isolement de gènes ayant normalement une action dans la suppression tumorale et dont il sera alors possible de surveiller et de traiter les éventuels dysfonctionnements.

En particulier, l'isolement de ces gènes permet d'avoir recours à une thérapie génique de remplacement ou bien à la synthèse d'agents pharmacologiques, protéiques ou non protéiques, qui, directement ou indirectement, par leur action sur les promoteurs, induiront l'activation et l'expression de ces gènes, ou encore la synthèse d'agents pharmacologiques qui permettront de mimer l'effet physiologique de ces gènes suppresseurs.

L'objectif final est, soit d'inhiber la croissance tumorale, ou mieux, d'induire le processus apoptotique de ces cellules tumorales, c'est-à-dire de conduire les cellules tumorales à se "suicider".

La présente invention concerne la mise en évidence de gènes qui sont impliqués dans cette apoptose. En effet, chaque cellule possède en elle un programme de mort physiologique. Il s'agit également d'un processus physiologique qui est impliqué dans le développement afin de maintenir l'homéostasie du corps et de ne pas voir des proliférations cellulaires anormales s'établir, même si, au demeurant, elles n'ont pas de caractère malin.

L'un des gènes suppresseurs les plus importants impliqués dans l'apoptose est le gène p53. Dans sa fonction normale, ce gène contrôle la croissance cellulaire et le processus d'apoptose ; en particulier, c'est ce gène qui bloque la croissance cellulaire et qui doit induire le processus apoptotique afin d'éviter le développement d'un cancer. On a ainsi mis en évidence que des souris nullizygotes pour le p53 étaient beaucoup plus sensibles à la formation de tumeurs. On a également mis en évidence le fait que, dans les cancers, le gène p53 était très souvent altéré et conduisait à la production de protéines incapables de véhiculer le message d'apoptose.

C'est cette particularité qui a été mise en oeuvre dans le cadre de la présente invention.

En effet, la présente invention repose sur la constatation qu'il n'est pas possible, ou du moins qu'il parait très difficile, de mettre en place une thérapie de substitution directe lors d'un dysfonctionnement du gène p53. En effet, le p53 muté comme il l'est dans le cancer va annuler l'effet physiologique du p53 normal.

Il a donc fallu renoncer, du moins dans un premier temps, à une thérapie de substitution agissant directement au niveau de p53.

La présente invention s'est donc attachée à étudier les gènes situés en aval de p53 afin de "bipasser" la difficulté évoquée précédemment.

Afin d'isoler les gènes activés ou inhibés par le p53 normal (wild-type p53) on a effectué un ratissage global de l'expression des gènes dans une cellule induite en apoptose et dans la même cellule maligne, plus particulièrement dans une cellule exprimant le p53 normal dans sa fonction et dans une cellule exprimant le p53 muté dont la fonction est oncogénique. La comparaison des gènes exprimés (ARN messagers exprimés dans les deux types de cellule) a permis de mettre en évidence des gènes exprimés différentiellement, c'est-à-dire exprimés dans l'une des cellules alors qu'ils ne le sont pas dans l'autre (les gènes peuvent être activés ou inhibés).

On en déduit aisément que ces gènes sont impliqués dans le processus de cancérisation, dans un cas par leur absence, et, dans l'autre cas, par leur présence.

Pour cette étude différentielle, la méthode utilisée est la méthode décrite en 1992 par Liang et Pardee (Differential display of eucaryotic mRNA by mean of a polymerase chaine reaction).

Jusqu'à présent, l'isolement des gènes impliqués dans la suppression étan effectué soit par clonage positionnel, soit par l'emploi des doubles hybrides. La première méthode permettait, par un calcul statistique, de calculer la plus haute probabilité où pouvait se localiser, au niveau chromosomique, un gène suppresseur candidat pour un type bien particulier de cancer, surtout ceux d'origines familiales. Le système de doubles hybrides permet d'isoler une à une les protéines qui-interagissent avec un gène donné.

L'approche du problème selon la présente invention a permis d'isoler des séquences directement reliées à une fonction. Dès lors, au contraire du séquencage aléatoire des EST, les séquences sont des séquences dont la fonction est connue et qui sont impliquées dans le processus d'apoptose induit par le gène suppresseur p53.

De façon plus précise, cette méthode a été utilisée sur un modèle cellulaire décrit par Moshe Oren, il s'agit de cellules myeloïdes tumorales de souris qui ont été transfectées par un mutant stable du gène p53, En fait, l'expression de ce gène est thermosensible, c'est-à-dire que dans des conditions de culture cellulaire à 37°C la protéine produite est une protéine mutée, c'est-à-dire qu'elle ne peut jouer le rôle de suppresseur de tumeur et donc que la lignée cellulaire correspondante se développe sous forme de cellule maligne, alors qu'à la température de 32°C la protéine p53 exprimée, comme la protéine naturelle, est capable de jouer le rôle de suppresseur et empêche la lignée cellulaire correspondante de devenir maligne.

Cette étude systématique a permis de mettre en évidence les gènes impliqués dans la cascade de suppression induite par p53.

C'est pourquoi les inventeurs ont mis en évidence de nouvelles séquences et les gènes les comportant ainsi que l'utilisation de ces séquences, tant au niveau du diagnostic qu'au niveau de la thérapie, de même que pour la réalisation de modèles destinés à tester des produits anti-cancéreux.

Leurs travaux concernent les séquences IND.SEQ 1 à 10 et leur application notamment dans la suppression du cancer ainsi que dans le suivi thérapeutique.

De plus, ils ont également identifié un gène humain impliqué dans la cascade de suppression induite par p53 ainsi que l'utilisation des séquences de ce gène, tant au niveau du diagnostic qu'au niveau de la thérapie, de même que pour la réalisation de modèles destinés à tester des produits anti-cancéreux ainsi que leur application à titre d'agent antiviral.

Leurs travaux ont ainsi porté sur une séquence selon l'IND.SEQ 11 correspondant au gène TSAP. 3 humain ou HUMSIAH (Human Homologue of the Drosophila seven in absentia gene), et leur application notamment dans la suppression du cancer ainsi que dans le suivi thérapeutique.

Par "séquences 1 à 11", il est entendu aussi bien la séquence nucléotidique correspondant au gène entier que des fragments de ce gène, notamment lorsqu'ils codent pour une protéine équivalente comme cela sera décrit ci-après.

Les séquences nucléotidiques peuvent être aussi bien de l'ADN que de l'ARN ou des séquences dans lesquelles certains des nucléotides sont non naturels, soit pour améliorer leurs propriétés pharmacologiques, soit pour permettre leur identification.

Les séquences mentionnées en (b) (pour les IND.SEQ 1 à 11) sont essentiellement les séquences complémentaires totales ou partielles (notamment pour les cas évoqués précédemment).

Les séquences (a) et (b) (pour les IND.SEQ 1 à 10) permettent non seulement l'accès au gène murin dont elles sont issues, mais également aux gènes humains correspondant par homologie.

Enfin, lorsque lesdits gènes codent pour une protéine, la présente invention s'applique également aux séquences codant pour la même protéine, compte tenu de la dégénérescence du code génétique, mais également pour les protéines équivalentes, c'est-à-dire produisant les mêmes effets, notamment les protéines délétées et/ou ayant subi des mutations ponctuelles.

Les séquences ci-dessus mentionnées sont plus particulièrement les séquences qui sont induites ou inhibées lors de l'apoptose cellulaire, notamment celles induites par p53.

Lesdits gènes sont regroupés en TSAP ou "Tumor Supprcssor Activated Pathway" et dénommés de TSAP 1 à TSAP 8 et TSAP 3 humain, correspondant aux IND.SEQ 1 à 8 et 11 (HUMSIAH) respectivement, et en TSIP ou "Tumor Suppressor Inhibited Pathway" et dénommés TSIP 1 et TSIP 2. correspondant aux IND.SEQ 9 et 10.

Les caractéristiques des séquences correspondent aux IND.SEQ 1 à 10 sont rassemblées dans le tableau ci-annexé.

Les séquences nucléotidiques correspondant aux gènes TSAP (y compris le TSAP 3 humain ou HUMSIAH), sont des séquences exprimées lors du processus d'apoptose alors que lorsqu'ils ne sont pas exprimés le processus d'oncogénèse se poursuit. Il est donc intéressant :
- de détecter toute anomalie dans le gène correspondant, laquelle peut conduire à une plus grande susceptibilité à l'oncogénèse, et
- de pouvoir prévoir une thérapie de remplacement.

Il faut d'ailleurs rappeler que ces gènes peuvent intervenir dans d'autres processus que les processus oncogènes ; en effet, p53 est en quelque sorte le gardien de l'intégrité du génome, dans ces conditions les gènes TSAP ou TSIP sont sans doute également impliqués dans cette fonction de contrôle, c'est donc l'ensemble des altérations possibles du génome qui peuvent être redevables de la détection et de la thérapie précédente. Au contraire, les gènes TSIP sont exprimés lors de l'oncogénèse et non lors de l'apoptose, il est donc là aussi intéressant de détecter l'éventuelle anomalie des TSIP et également de prévoir une thérapie d'inhibition/blocage.

La thérapie de remplacement pourra être effectuée par thérapie génique, c'est-à-dire en introduisant le gène TSAP avec les éléments qui permettent son expression in vivo. Les principes de la thérapie génique sont connus. On peut utiliser des vecteurs particuliers, viraux ou non viraux, par exemple des adénovirus, rétrovirus, virus herpès ou poxvirus. La plupart du temps ces vecteurs sont utilisés sous forme défectifs qui serviront de véhicules d'expression de TSAP avec ou sans intégration. Les vecteurs peuvent être également synthétiques, c'est-à-dire mimer des séquences virales, ou bien être constitués par de l'ADN ou de l'ARN nu selon la technique développée notamment par la société VICAL.

Dans la plupart des cas, il faudra prévoir des éléments de ciblage assurant une expression spécifique des tissus ou organes, en effet, il n'est pas possible d'envisager d'activer un phénoméne d'apoptose incontrôlé.

Les systèmes d'expression pour produire des protéines peuvent être aussi bien des systèmes eucaryotes tels que les vecteurs précédents que des systèmes procaryotes dans des cellules de bactéries.

La mise en évidence de l'implication de plusieurs gènes dans l'apoptose est décrite ; ainsi la surexpression de l'un des gènes par thérapie génique peut, pour certains d'entre eux, ne conduire à l'apoptose que les cellules dans lesquelles s'expriment déjà d'autres gènes déréglés, c'est-à-dire des cellules malignes.

La présente invention concerne également, à titre de médicament, un composé assurant l'inhibition de l'expression cellulaire de SEQ ID N° 10 telle que décrite précédemment lorsqu'elle est inhibée lors de l'apoptose cellulaire, à savoir TSIP 2.

Il est, par exemple, possible de prévoir d'autres approches que la thérapie génique, notamment l'utilisation de séquences nucléotidiques en stratégie sens ou antisens, c'est-à-dire pouvant bloquer l'expression de TSIP2 (SEQ ID N° 10).

On peut également prévoir une stratégie de remplacement directe par apport de d'anticorps inhibiteurs correspondant à TSIP2 (SEQ ID N°10).

Enfin, il est possible de prévoir l'utilisation de molécules non protéiques dont l'activité sera d'inhiber TSIP2 ou bien de bloquer l'action de son produit d'expression.

Ces produits peuvent être aisément testés sur les cellules modifiées qui sont décrites dans les exemples en introduisant les produits à tester dans la culture cellulaire et en détectant l'apparition du phénomène apoptotique. Dans les stratégies à ADN, ARN ou protéique les produits sont bien entendu élaborés en fonction des séquences qui sont décrites.

La présente invention concerne en particulier l'utilisation des médicaments précédents en tant qu'agent anti-cancéreux.

Le produit du gène TSAP 3 humain (HUMSIAH) est également utile comme agent antiviral, comme cela apparaitra à la lecture de l'exemple 2.

La présente invention concerne également à titre d'agent de diagnostic pour la détermination de la prédisposition au cancer, tout ou partie de SEQ ID N° 10 à utiliser comme sonde nucléotidique ou comme amorce d'amplification, mais également à titre d'agent de diagnostic pour la détermination de la prédisposition au cancer un antigène correspondant à tout ou partie des protéines codées par la séquence selon l'invention ou les anticorps, notamment les anticorps monoclonaux, correspondants, éventuellement après culture.

Les méthodes de diagnostic sont connues, il peur s'agir, par exemple, de techniques de microséquencage des parties variables après isolement et amplification éventuelle ou des méthodes de détection type RFLP ou d'amplification simple notamment. Les techniques différentielles peuvent, en particulier, permettre de mettre en évidence l'écart entre le TSAP ou TSIP normal et anormal.

Le gène TSAP 3 humain (HUMSIAH) peut être isolé, notamment, en utilisant la méthode PCR ou d'autres méthodes d'amplification en mettant à profit la structure du gène. Il est également possible de synthétiser ce gène par morceau, si nécessaire.

Enfin, les inventeurs ont réalisé un perfectionnement à la méthode de Liang et Pardee (1) caractérisé en ce que dans l'amplification par PCR on effectue une diminution en palier ("touch down") tel que décrit dans Don et al. (2).

D'autres caractéristiques de l'invention apparaitront à la lecture des exemples ci-après faite notamment en se référant aux figures suivantes :

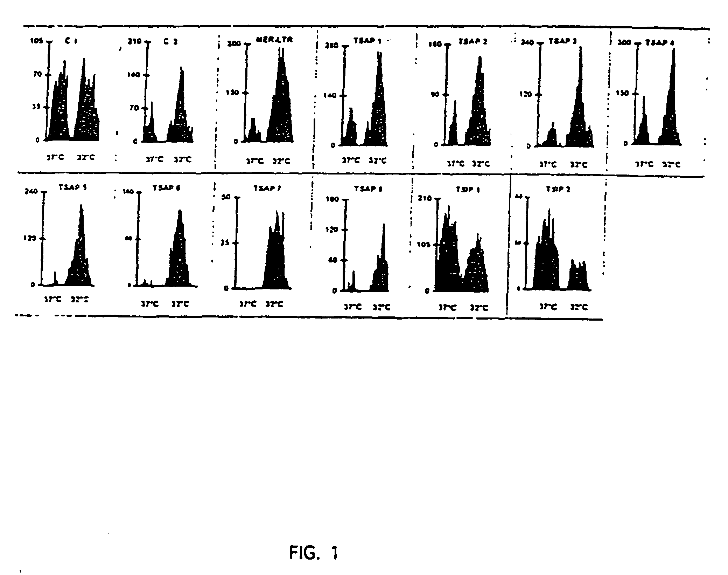

- Figure 1 - Quantification de l'expression différentielle des ARNm utilisant l'imageur 1200 β. Hybridation aux ARNm dérivés des cellules LTR6 à 37°C et des cellules LTR6 après 4 heures à 32°C. Les nombres en ordonnées de 0 à 500 correspondent au comptage détecté par 0.15 mm et sont proportionnels au signal d'hybridation.
   C1 : ARNm exprimé également en utilisant un clone sans expression différentielle :
   C2 : contrôle positif utilisant la Cyclinc C et montrant l'induction des ARNm correspondant à 33°C ;
   MER-LTR : montre l'induction de cette séquence à 32°C ;
   TSAP 1 à TSAP 8 : expression différentielle des 8 ARNm activés dans les 4 premières heures suivant l'induction de l'apoptose ;
   TSIP 1 et TSIP 2 : expression différentielle des 2 ARNm inhibés dans les 4 premières heures suivant l'induction de l'apoptose.

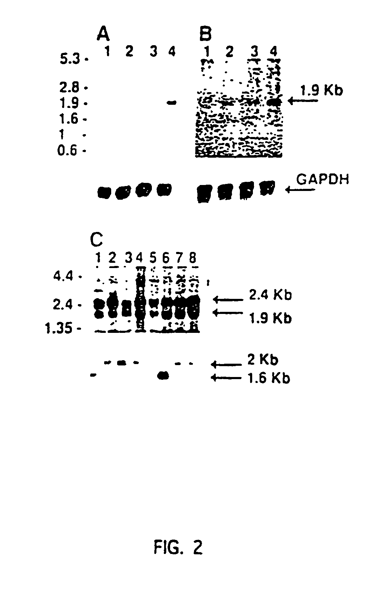

- Figure 2 - Analyse Northern blot.
   A : hybridation avec la sonde TSAP 3 ;
   B : hybridation avec la sonde siah 1b de souris ;
   lignes 1 et 2 : ARNm polyA⁺ de cellules leucémiques myéloïdes M1 (clone S6) cultivées à 37°C et 32°C respectivement ;
   lignes 3 et 4 : ARNm polyA⁺ de cellules LTR6 cultivées à 37°C et 32°C respectivement ;
   la flèche indique l'expression différentielle du transcrit 1.9 kb de TSAP 3 - siah 1 b de souris ;
   panneaux inférieurs : GAPDH ;
   C : distribution tissulaire utilisant TSAP 3 comme sonde ;
   1 : coeur, 2 : cerveau, 3 : rate, 4 : poumon, 5 : foie, 6 : muscle du squelette, 7 : rein, 8 : testicule ;
   les flèches indiquent les transcrits de 1,9 et 2,4 kb ;
   panneau inférieur : β-actine.

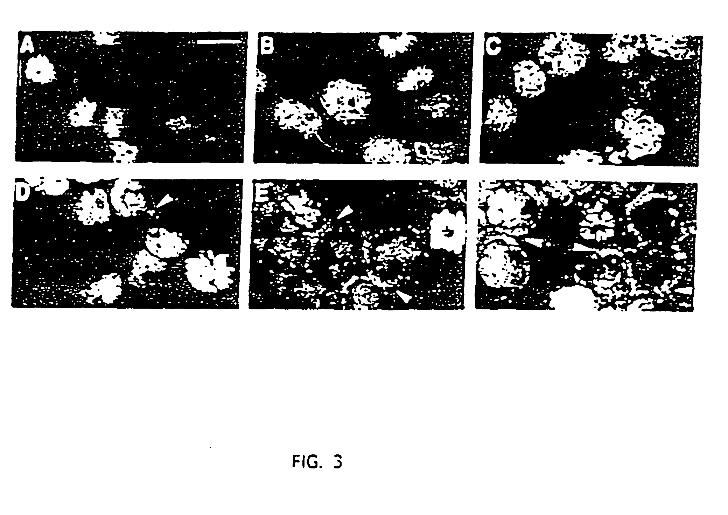

- Figure 3 - Analyse de l'hybridation in situ avec la sonde TSAP 3 ;
   A : cellules M1 incubées pendant 4 heures à 32°C et hybridées avec une sonde antisens TSAP 3 ;
   B : cellules LTR6 incubées pendant 4 heures à 32°C et hybridées avec une sonde sens TSAP 3 ;
   C : cellules LTR6 incubées à 37°C et hybridées avec une sonde antisens TSAP 3 ;
   D à F : cellules LTR6 cultivées à 32°C pendant respectivement 1, 2 et 4 heures et hybridées à une sonde antisens TSAP 3 ;
   la barre dans le panneau A : 10 µm ;
   les flèches indiquent l'accumulation des ARNm TSAP 3 dans le cytoplasme.
- Figure 4 - Comparaison entre la séquence d'ADNc de TSAP 1 et la séquence nucléotidique correspondant à la phospholipose C béta 4 de rat.
- Figure 5 - Comparaison entre la séquence d'ADNc de TSAP 2 et la séquence nucléotidique correspondant à la protéine digitée au zinc (ZFM 1) localisée dans le locus Multiple Endocrine Neoplasia (MEN 1).
- Figure 6 - Comparaison entre la séquence d'ADNc de TSAP 3 et la séquence nucléotidique correspondant au gène Drosophila seven in absentia (sina).
- Figure 7 - Comparaison entre le produit des gènes sina de différentes espèces, humain (HUMSIAH), murin (MMSIAH 1B) et de drosophile (DROSINA).
- Figure 8 - Comparaison entre la séquence d'ADNc de TSIP 2 et la séquence d'ADNc du transcript S182 murin du gène AD3 impliqué dans la maladie d'Alzheimer.

### MATERIELS ET METHODES

### Cultures cellulaires

Cellules de leucémie myéloide M1 (clone S6) et cellules M1 transfectées de façon stable avec un mutant sensible à la température val 135 p53 (LTR6) (3).

Ces cellules sont cultivées sur milieu RPMI 1640 avec 10 % FCS à 5 % de CO₂ à 37°C. Pour la modification de la température, les cultures sont placées dans un second incubateur à 32°C. Pour tous les essais effectués dans cette étude, les cellules sont testées après 12 et 24 heures pour la présence d'apoptose.

### Etude des ADNc différentiels

Pour effectuer les tests dans des conditions expérimentales standards et pour obtenir une reproductivité totale des résultats, les modifications suivantes au protocole d'origine (1) ont été effectuées.

On utilise toujours des ARNm polyA⁺ purifiés deux fois sur colonne d'oligodT utilisant Fast Track (Invitrogen, San Diega CA). Après transcription réverse (M-MLV Reverse Transcriptase, Gibco BRL) sur 0,05 µg de polyA⁺ utilisant 20 µM de chacun des dNTP (Boehringer-Mannheim), aucun dNTP additionné n'est ajouté au mélange de PCR final. Un "hot start" à 94°C pendant 5 minutes est effectué avant la PCR (GeneAmp PCR system 9600 Perkin Elmer Cetus). Les échantillons sont refroidis rapidement sur de l'eau glacée. Un "touch down" (2) de 10 cycles de 50°C à 40°C est effectué (94°C 30 secondes - 50°C 1 minute - 72°C 30 secondes), suivi par 35 cycles (94°C 30 secondes - 40°C 1 minute - 72°C 30 secondes) et une extension finale de 5 minutes à 72°C. Les produits de la PCR sont séparés sur gels de polyacrylamide à 6 % non dénaturant (4). Les gels sont exposés sans séchage. Chaque présentation différentielle est effectuée en comparant M1S6 et LTR6 à 37°C et après 4 heures d'incubation des deux lignées cellulaires à 32°C.

La procédure de présentation différentielle est répétée dans 3 expériences différentes pour confirmer une parfaite reproductibilité.

Les bandes exprimées différentiellement sont découpées à partir du gel, éluées et réamplifiées (1). Les produits de PCR sont sous-clonés en utilisant le système TA-cloning (Invitrogen, San Diego CA) en suivant les indications fournies.

Pour chaque réaction de ligation, 10 clones recombinants sont séquencés en utilisant le système automatique ABI.

### Extraction des ARN, analyses et sondes Northern blots

L'ARN total est extrait avec du Trizol (Life Technologies). Les ARN polyA⁺ sont préparés en utilisant le kit OligotexdT (Qjagen, CA). 30 µg de l'ARN total ou 2 µg d'ARN polyA⁺ sont séparés sur agarose 1 %/1 x MOPS / 2 % gel de formaldéhyde, transférés sur membrane de nylon (Hybond N+, Appligène, France) comme cela a été décrit précédemment (5). Les Northern blots sont hybridés avec des sondes marqués au P32 sur les inserts TSAP et TSIP et lavés comme décrit précédemment (5). Pour vérifier l'induction de la fonction du p53 sauvage, les Northern blots sont hybrides avec une sonde cycline G (6). A titre de contrôle pour la quantité d'ARNm chargée, les blots sont hybridés avec une sonde GAPDH. Différents Northern blots (Clontech CA) sont utilisés dans des conditions identiques et hybrides pour le contrôle avec une sonde β-actine. Les produits de RT-PCR pour LTR6 sont amplifiés en utilisant les_amorces siah 1b suivantes : 5'CAGTAAACCACTGAAAAACC3' et 5'CAAACCAAACCAAAACCAC3'. Le produit de PCR sous-cloné est utilisé comme sonde contrôle de siah 1b. Les Northern blots sont exposés pendant 10 jours à - 80°C.

### Slot blots

La reproductibilité des résultats obtenus par les analyses Northern blot. Les blots sont préparés (Bio-Rad, Hercules CA) en plaçant les produits de PCR (200 ng de Zeta-Probe Blotting Membranes, Bio-Rad, suivant les instructions du fabricant) de clones TSAP et hybridés avec une sonde ADNc marquée au P32 (Superscript II Gibco-BRL, Life Technologies) correspondant à l'ARN des cellules LTR6 incubées à 37°C et ensuite 4 heures à 32°C. Le produit de PCR du clone contenant la cycline G est également déposé sur les membranes et utilisé comme contrôle positif. Les Slot blots sont exposés une nuit à - 80°C.

### Analyse quantitative des images

Celle-ci est effectuée en utilisant un imageur 1200 β (Biospace Instruments, Paris, France) sur les deux Northern blots (pour TSIP 1 et TSIP 2) et sur les Slot blots pour tous les contrôles ADNsc et TSAP 1 à 8. Pour l'analyse quantitative représentée dans les graphiques de la figure 1 on soustrait un nombre constant de chaque pic. Cette constante est calculée en mesurant la valeur moyenne du bruit de fond dans les slots qui ne contiennent pas d'ADNc. Les résultats du β imageur ont été obtenus en comptant les slot blots une nuit et en les confirmant par autoradiographie avec des temps variables d'exposition. Ces autoradiogrammes montrent les mêmes variations qualitatives relatives entre les activités à 32°C et à 37°C que les mesures effectuées avec le β imageur.

### Hybridation in situ (7, 8)

Les cellules sont lavées 3 fois dans un tampon phosphate salin (PBS) "cytospinned" et fixées par du paraformaldéhyde à 4 % dans PBS pendant 10 minutes puis conservées dans l'éthanol à 70 %. Des transcrits d'ARN marqués à la digoxigénine-11-urédine-5'-triphosphate (DIG) et à la biotine-11-UTP de TSAP 3 sont utilisés dans les analyses suivant la procédure décrite précédemment (Boehringer-Mannheim). Pour la détection des souches marquées à la digoxigénine hybridée les tranches sont incubées dans SAD-10 (10 nm d'anticorps anti-DIG de mouton marqués à l'or à 1/1000 de dilution, Biocell UK). L'analyse est effectuée en utilisant de la microscopie à laser confocal.

### EXEMPLE 1

L'étude différentielle des ADNc par la méthode de Liang et Pardee permet de disposer d'un outil très puissant et efficace pour détecter les variations dans l'expression des gènes. Néanmoins, il a fallu modifier le protocole original comme cela a été indiqué précédemment afin d'écarter certains problèmes de reproductibilité observés lorsque l'on applique la méthode telle qu'elle est décrite à l'origine.

On a pu mettre en évidence une reproductibilité totale lorsque dans la méthode PCR on introduit un "hot start" suivi par un "touch down".

Les bandes exprimées différentiellement après isolement et réamplification sont néanmoins souvent contaminées par des bandes provenant des ARN qui migrent dans les régions voisines de l'ADNc, si l'on utilise directement ces sondes sur des Northern blots ceci conduit à des erreurs. On a donc sous-cloné les produits de seconde PCR et fait effectuer les analyses des Northern blots utilisés à défaut de recombinant à sonde simple. Le séquençage systématique d'au moins 10 sous-clones recombinants pour chaque bande sélectionnée a montré qu'il était très efficace pour sélectionner les clones d'intérêt.

Le gène p53 est, dans l'état actuel de nos connaissances, le suppresseur tumoral qui est muté dans le plus grand nombre de cancers d'origines très diverses, et l'utilisation du mutant sensible à la température val-135 p53 s'est déjà montrée précédemment fournir des informations très importantes concernant le fonctionnement du p53. sauvage en induisant, soit l'arrêt de la croissance cellulaire en phase G-1, soit l'initiation du programme de mort cellulaire.

Jusqu'à maintenant, les voies moléculaires en amont et en aval de p53 et qui conduisent à la suppression tumorale étaient encore peu claires.

Jusqu'à maintenant un certain nombre de gènes en aval de p53 ont été identifiés, il s'agit notamment de gadd 45, mdm 2, mck, "Mouse endogenous retrovirus" LTR, p21-waf et Cycline G.

La présente invention a permis de mettre en évidence l'existence de 11 gènes qui sont exprimés différentiellement dans les cellules exprimant le p53 sous sa forme suppresseur actif ou bien dans des cellules tumorales exprimant le gène p53 non actif.

La figure 1 montre la quantification des signaux d'hybridation correspondant à l'expression différentielle de 8 de ces gènes qui sont activés à 32°C, c'est-à-dire dans lesquels la fonction de p53 sauvage est activée et conduit donc à l'apoptose des cellules, ces gènes qui sont activés seront dénommés ci-après TSAP (pour Tumor Suppressor Activated Pathway), par contre on constate que dans deux expériences 2 gènes exprimés à 37°C sont en partie inhibés à 32°C, ce qui impliquerait qu'ils sont inhibés durant la mort cellulaire programmée, ces gènes ont été dénommés TSIP (pour Tumor Suppressor Inhibited Pathway).

L'analyse des homologies des différentes séquences activées de TSAP 1 à TSAP 3 a montré qu'il s'agissait là de gènes déjà connus. Par contre, les autres ADNc TSAP 4 à TSAP 8 ne montrent aucune homologie significative avec des gènes connus.

Pour l'ADNc TSIP 1 qui est inhibé dans son expression pendant l'apoptose, il ne montre aucune homologie avec des gènes connus.

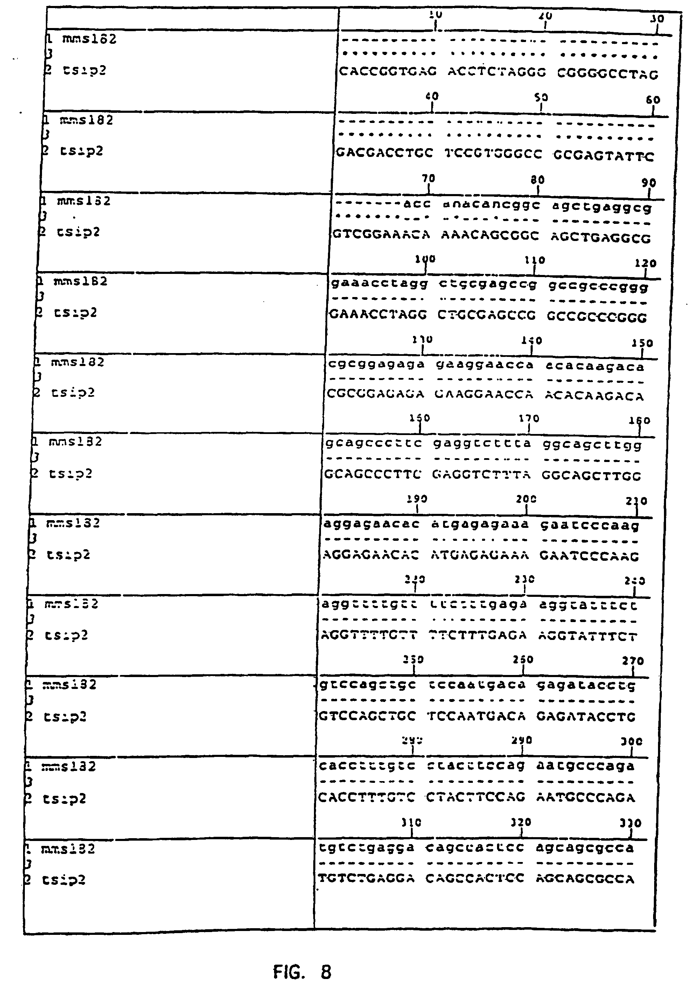

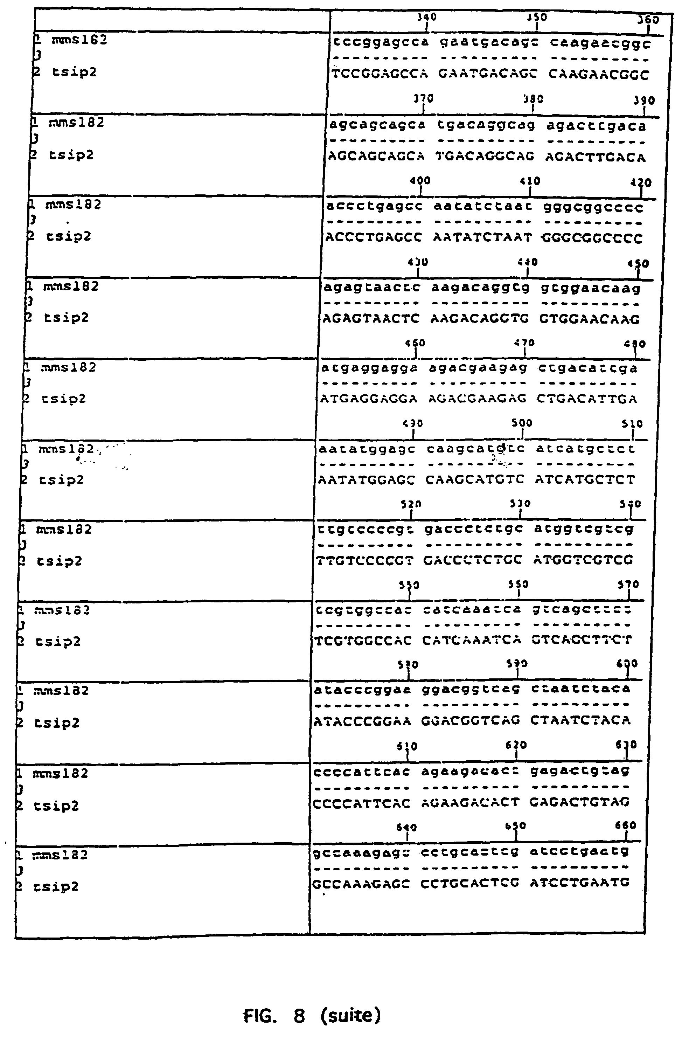

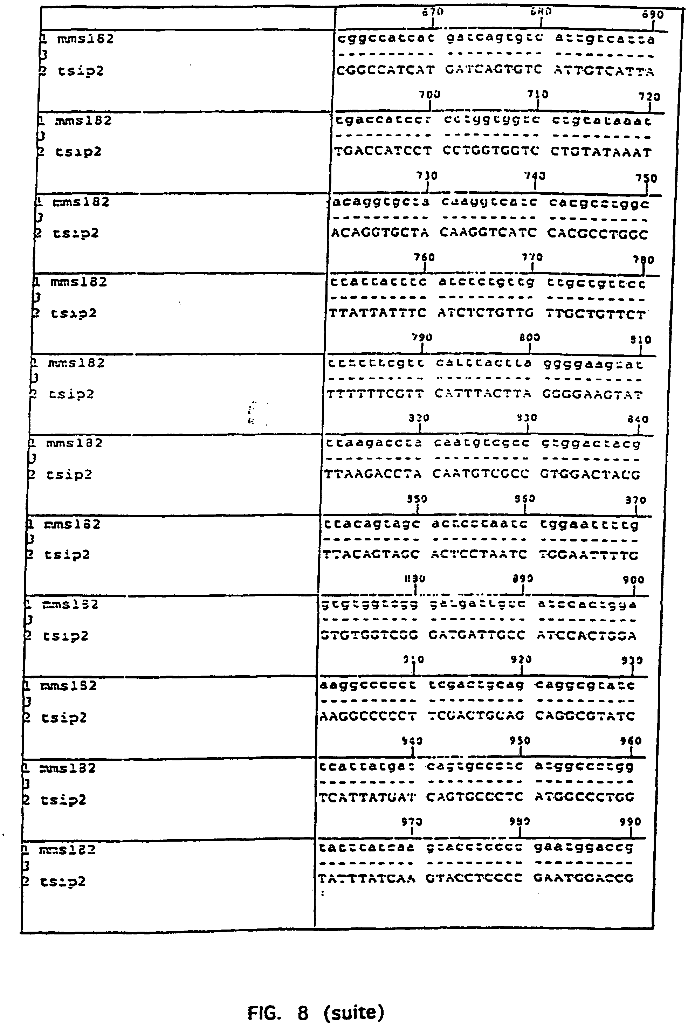

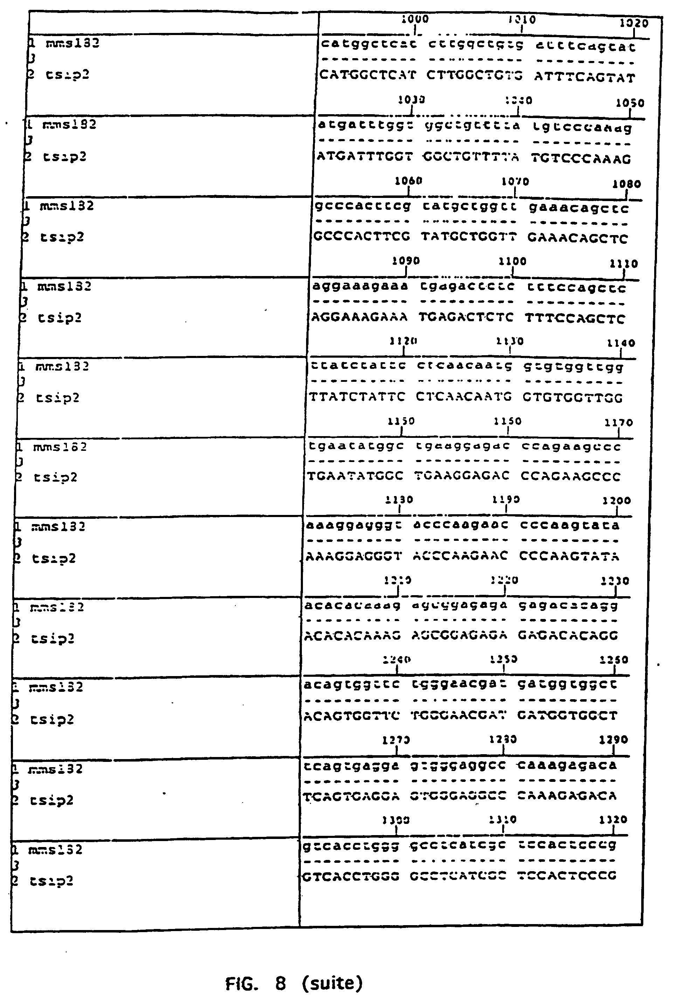

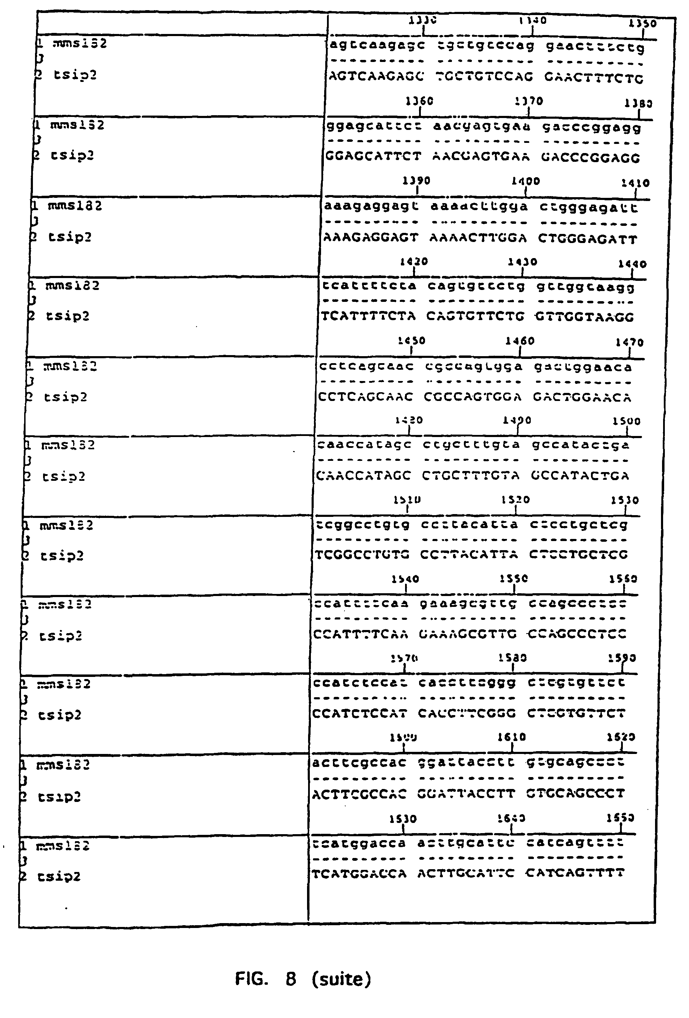

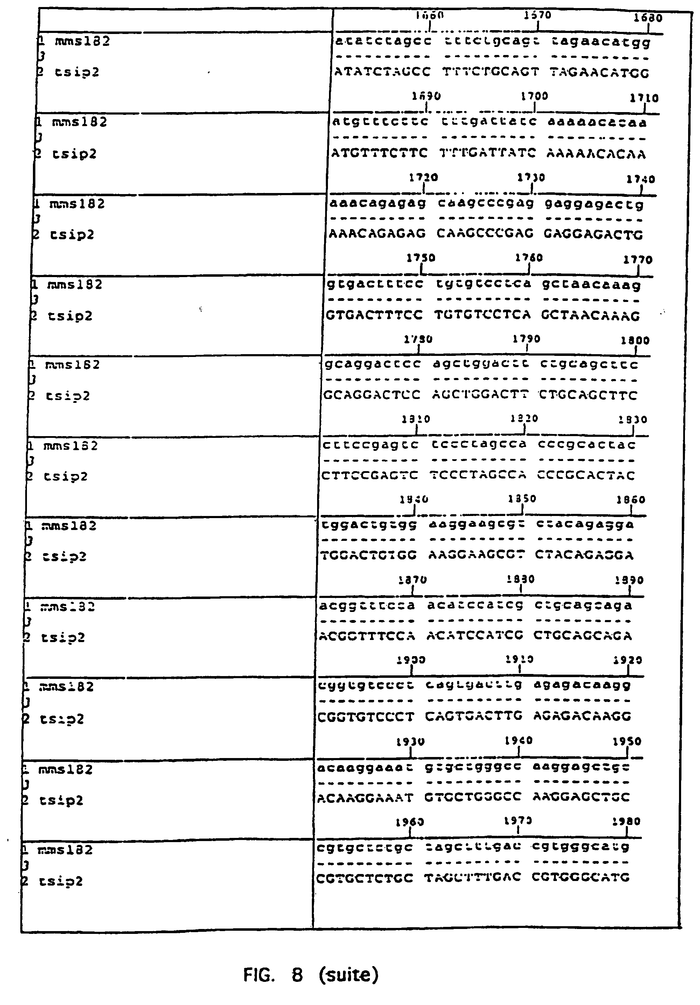

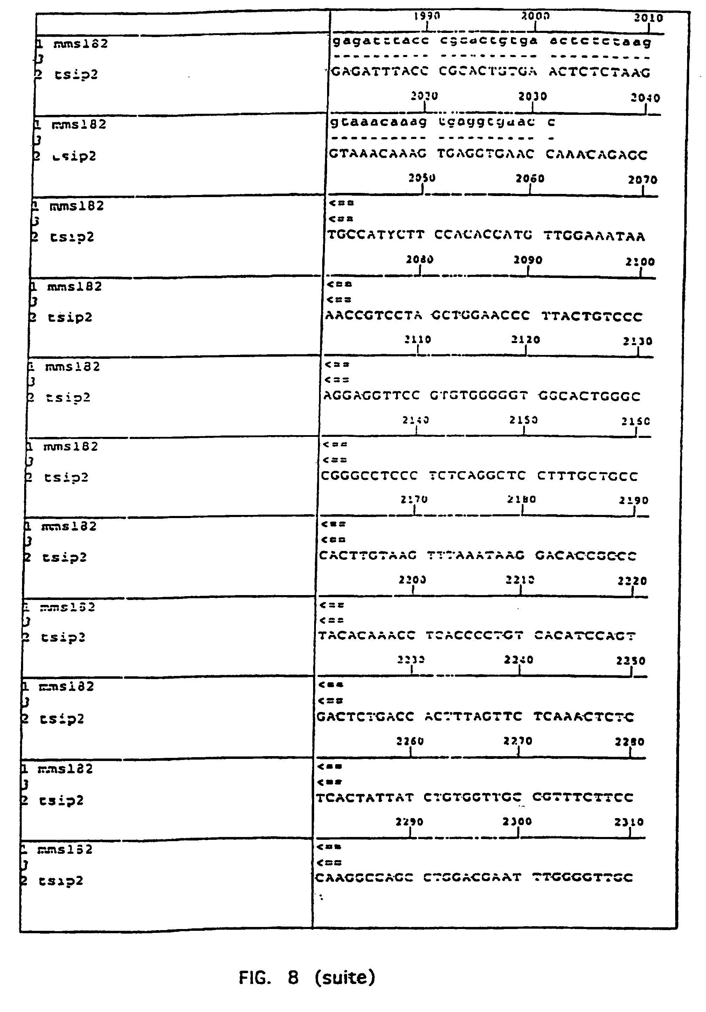

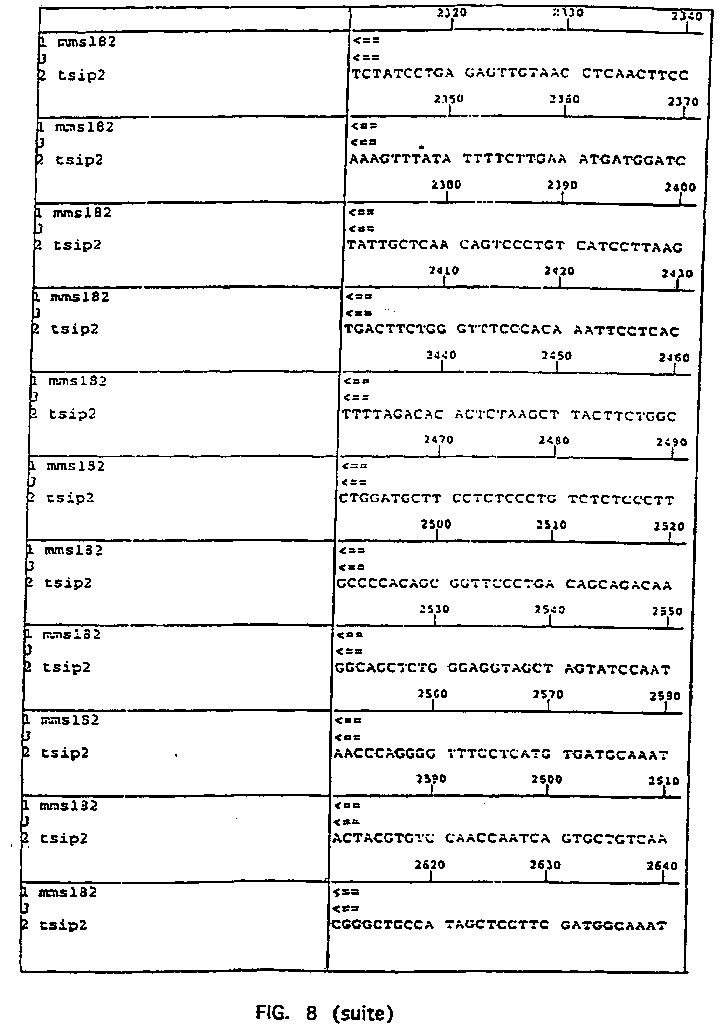

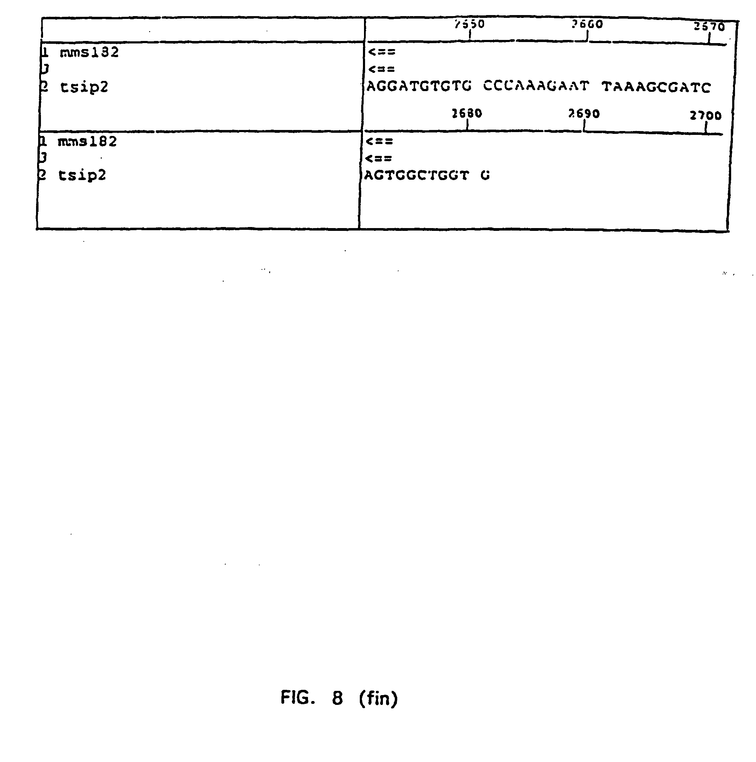

Pour l'ADNc TSIP 2 qui est également inhibé dans son expression pendant l'apoptose, il montre une grande homologie avec le transcript S182 du gène AD3 impliqué dans les voies métaboliques de la maladie d'Alzheimer (Sherrington et al.) (figure 8).

Par conséquent, il est possible d'agir sur les voies métaboliques de la maladie d'Alzheimer en agissant sur les voies métaboliques p53 dépendantes.

La présente invention a donc également pour objet, à titre de médicament, un composé assurant l'expression cellulaire de TSIP 2 destiné au traitement de la maladie d'Alzheimer ainsi qu'à titre d'agent de diagnostic pour la détermination de la prédisposition à la maladie d'Alzheimer, tout ou partie de la séquence de TSIP 2 à utiliser comme sonde nucléotidique ou comme amorce d'amplification ainsi qu'un antigène correspondant à tout ou partie des protéines codées par TSIP 2 ou les anticorps, notamment les anticorps monoclonaux correspondants, éventuellement après culture.

L'hypothèse que l'on peut faire sur ces gènes inhibés dans leur expression par le p53 sauvage est qu'ils peuvent coder pour des séquences oncogéniques qui seraient régulées en aval du processus de suppression tumorale ou encore qu'il s'agit de protéines de structure ou du cytosquelette pour lesquelles la régulation en aval de l'expression est concomitante de la mort cellulaire par apoptose.

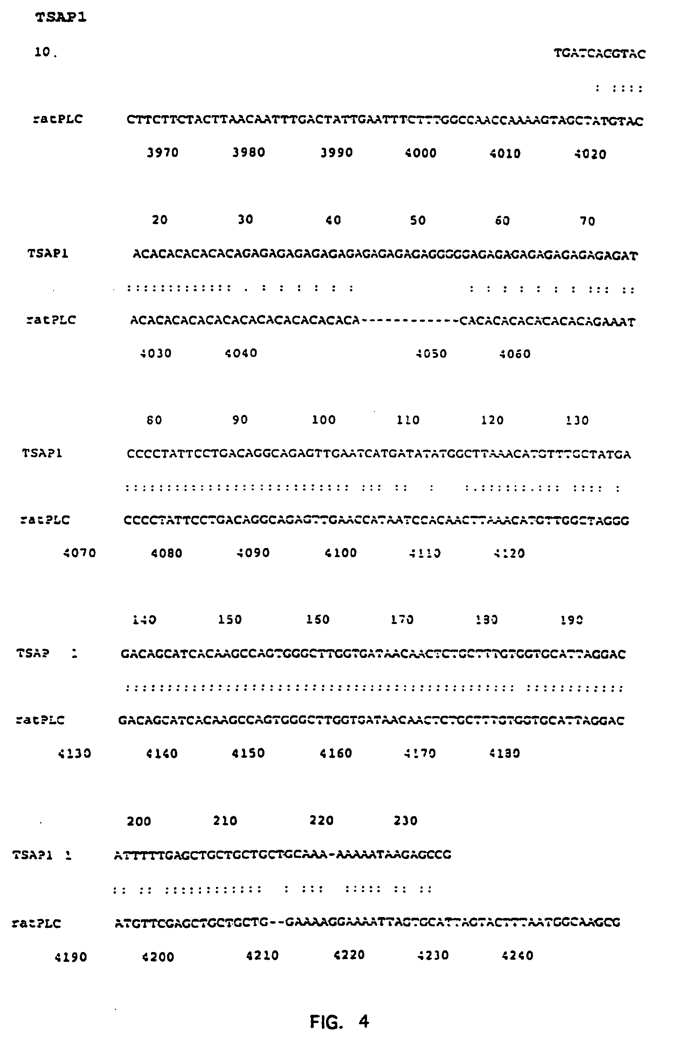

TSAP 1 est homologue à la phospholipase C bêta 4 de rat. La séquence de TSAP 1 présente 100 % d'identité avec la PLC entre les nucléotides 3967 et 3985 ; 82 % entre les nucléotides 3986 et 4116 et 85 % entre les nucléotides 4070 et 4220 (figure 4). La PLC est connue pour être impliquée dans la voie de signalisation des récepteurs de la tyrosine-kinase, et pour catalyser l'hydrolyse du phosphatidylinositol-4,5-biphosphate en diacylglycérol et inositol-1,4,5-triphosphate. Toutefois, la présente étude suggère que la PLC est une cible en aval dans l'apoptose à médiation p53.

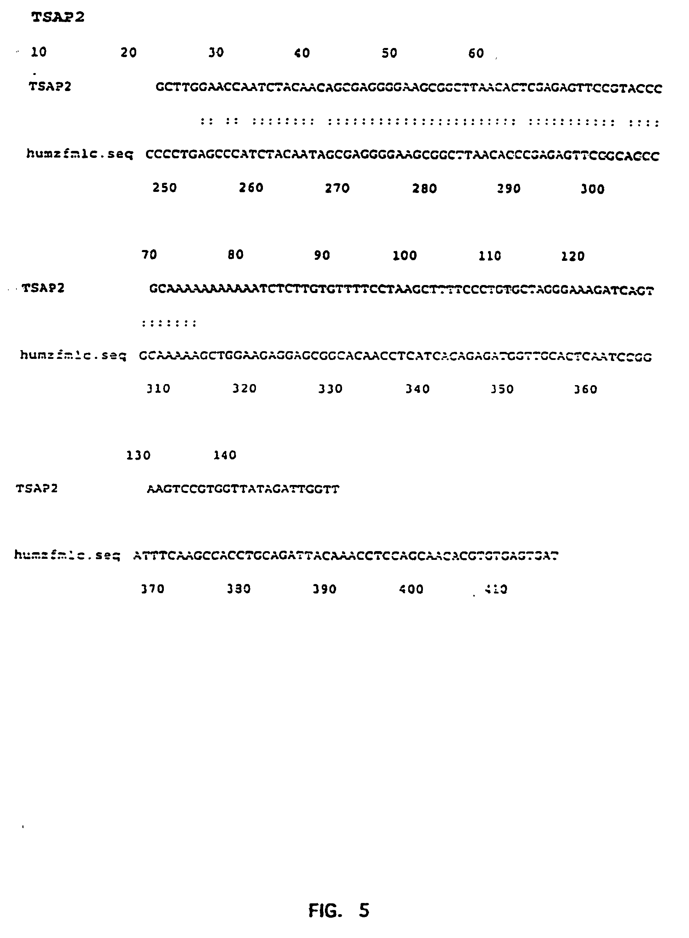

TSAP 2 montre des séquences conservées (92 % d'identité entre les nucléotides 259 et 299 ; 100 % d'identité entre les nucléotides 418 et 458 et 92 % d'identité entre les nucléotides 645 et 685) avec la protéine digitée au zinc (ZFM 1) qui est localisée dans le locus Multiple Endocrine Neoplasia (MEN 1) (figure 5). MEN 1 est un désordre dominant autosomal associé avec le développement de tumeurs affectant le lobe antérieur des glandes pituitaires et parathyroïdes et les cellules des ilots pancréatiques. Il est particulièrement intéressant d'avoir mis en évidence qu'à la fois ZFM et une isoenzyme de PLC sont colocalisés dans la même région chromosomique 11q13 contenant le gène de susceptibilité à MEN 1. Chez la souris, les régions homologues sont localisées sur le chromosome 19B. Le fait de trouver que TSAP 1 et TSAP 2 sont activés en réponse à p53 peut suggérer que ces gènes appartiennent à une voie de suppression des tumeurs plus globale et que p53 peut coopérer avec MEN 1.

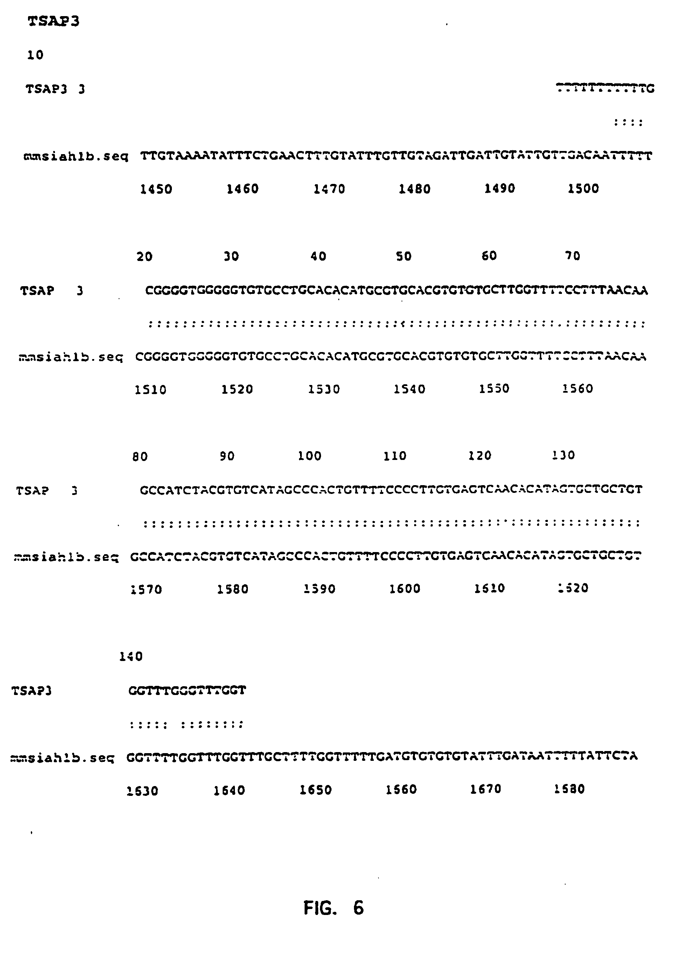

TSAP 3 est identique à Siah 1b. Ce gène est l'homologue chez les vertébrés du gène Drosophila seven in absentia (sina). Le clone décrit présente 94 % d'identité avec l'homologue murin (nucléotides 1496 à 1634) (figure 6). Par analyse Northern blot en utilisant une sonde TSAP 3, on a pu détecter une expression différentielle d'un messager de 1,9 kb de ce gène (figure 2A). Ceci est confirmé en utilisant une seconde sonde correspondant à la même région de la séquence siah 1b décrite (figure 2B). La figure 2C montre la distribution tissulaire de ce gène en utilisant une sonde TSAP 3 qui détecte à la fois l'ARNm de 1,9 et de 2,4 kb correspondant aux résultats mentionnés précédemment lorsqu'une sonde siah est utilisée. L'hybridation in situ montre que l'ARNm de TSAP 3 est induit rapidement 1 heure après l'induction de l'apoptose (figure 3D). Son expression augmente après 2 et 4 heures (figures 3E et 3F). Dans les cellules qui sont entrées en mitose aucun signal n'est détecté.

Carthew et Rubin ont montré que seven in absentia est nécessaire pour le développement de l'oeil de la drosophile. D'autre part, des mutants de ce gène dans la drosophile montrent un rôle beaucoup plus général dans le développement. L'homologue murin est subdivisé en deux groupes siah 1 et siah 2 et ces protéines montrent un degré de conservation tout à fait inhabituel par rapport à drosophila seven in absentia.

Nos résultats ont montré que TSAP 3 / siah 1b est activé dans le programme de mort cellulaire dans les cellules M1 induites par le gène suppresseur de tumeur p53. Comme ce gène code pour une protéine digitée au zinc nucléaire, il pourrait être un facteur de transcription régulateur qui est en aval du signal de p53. Les résultats montrent également un lien direct entre les gènes concernant le développement chez la drosophile et une voie majeure de suppression tumorale.

### EXEMPLE 2

En utilisant le fragment d'ADNc murin (TSAP 3), décrit ci-dessus, obtenu par analyse différentielle d'ARNm, on a constitué une sonde pour isoler un fragment de 1,1 kb d'une librairie d'ADNc humain qui ensuite a été expansé jusqu'à la région codante entière par une RACE-PCR.

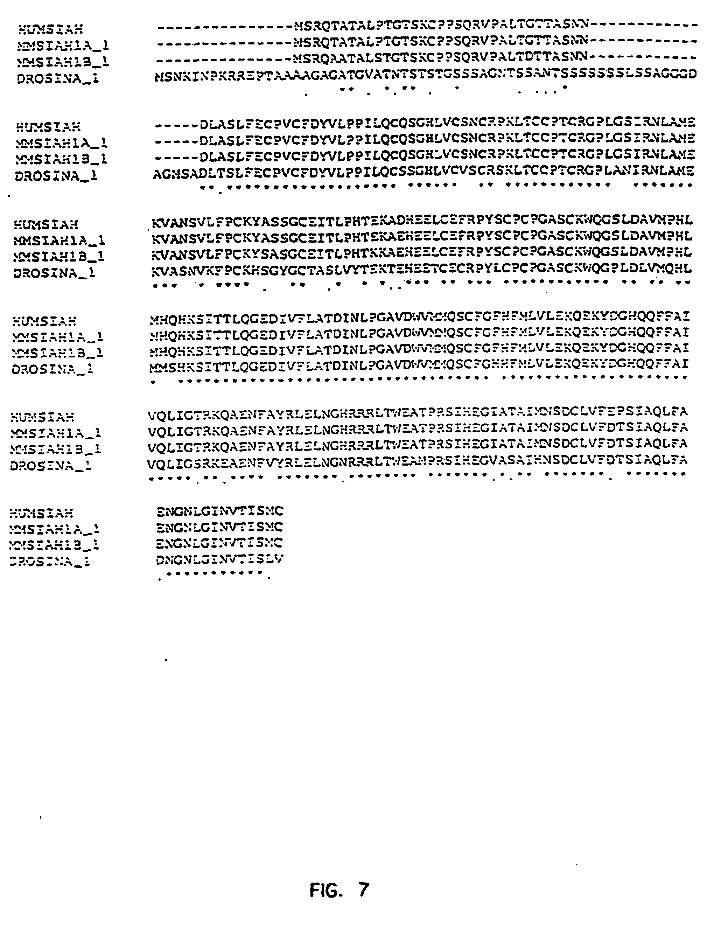

La figure 7 montre l'ADVc et la séquence d'acides aminés du gène humain sina (TSAP 3).

Cette séquence code une protéine de 282 amino-acides avec un motif digité au zinc C3HC4. Cette protéine présente également des analogies avec des protéines capables de se fixer sur l'ARN. La séquence en amino-acides est très conservée entre la Drosophile, la souris et le gène humain (figure 7).

La distribution tissulaire indique que le sina humain est exprimé de façon ubiquitaire et code pour un ARNm de 2,3 kb et, dans le placenta, il existe un transcrit additionnel de 2,5 kb.

En analysant des YAC du CEPH et des librairies BAC par PCR, en utilisant des amorces sina humains spécifiques, on a pu isoler 8 YAC (350-1000 kb) et 2 BAC (100 et 125 kb).

La fluorescence par hybridation in situ (FISH) utilisant les clones YAC et BAC montre que le seven in absentia est localisé sur le chromosome 16q12-13, c'est-à-dire dans une région contenant les gènes suppresseurs de tumeurs candidat dans différents cancers, notamment : cancer du sein (9), tumeur de Wilm's (10-12), syndrome de Laurence-Moon-Bard et-Biedl (13), syndrome de Beckwith-Wiederman (14).

Comme cela a été indiqué dans la demande de brevet français N° 95 15 146, on a trouvé que des transfectances stables de cellules M1 murines avec le mutant p53 sensible à la température montraient l'activation de seven in absentia après induction de l'apoptose à 32°C. Etant donné que le TSAP 3 murin a été isolé dans un modèle d'apoptose induit par le gène p53, il était logique d'approfondir l'analyse du gène TSAP3 (HUMSIAH) dans un modèle d'apoptose physiologique humain.

Ce modèle est décrit dans l'intestin où les cellules migrent du fond de la crypte vers la région apicale des vilosités où elles meurent par apoptose avant d'être larguées dans le lumen. Ces cellules en apoptose sont spécifiquement marquées par la technique TUNEL.

D'autre part, ces mêmes cellules sont positives par hybridation in situ pour le gène TSAP 3 (HUMSIAH) dans l'apoptose physiologique chez l'humain.

Enfin, afin d'investiguer l'implication du gène TSAP 3 humain dans la suppression des tumeurs, on a utilisé un modèle basé sur l'ensemble des gènes plutôt que sur un seul gène. Ce modèle repose sur les propriétés biologiques du parvovirus H-1.

Des recherches très complètes dans ce domaine ont montré sur les 20 dernières années que le parvovirus tue préférentiellement les cellules tumorales alors qu'il épargne leur contrepartie normale.

De façon à élaborer un modèle, on a fait l'hypothèse suivante : s'il était possible de sélectionner, à partir d'une tumeur qui soit sensible à l'effet cytopathique du parvovirus H-1, les cellules qui étaient résistantes, cette résistance pourrait être due à un changement de leur phénotype malin. Ceci a pu être démontré pour les cellules KS sélectionnées à partir des cellules érythro-leucémiques K562 humaines. Tandis que les cellules parentales K562 sont sensibles à l'effet cytopathique du pawovirus H-1, les cellules KS, elles, sont résistantes. Ces cellules résistantes réexpnment le type sauvage de p53 et ont un phénotype supprimé à la fois in vitro et in vivo.

Pour confirmer ces observations sur d'autres cellules, on a sélectionné, à partir d'un monoclone d'une leucémie monocytaire U937 humaine, les cellules filles US3 et US4. Ces clones sont résistants à l'effet cytopathique des parvovirus H-1 et montrent une réversion du phénotype malin in vivo. L'analyse de marqueurs de surface pour 20 cellules, indique qu'il n'y a pas de déplacement dans le stade de différentiation entre U937 et les clones US indiquant que la suppression du phénotype malin n'est pas due à une différentiation terminale.

Ni les cellules K562 ni les cellules U937 n'expriment p53. Par contraste aux cellules KS qui réexpriment p53, les cellules US3 et US4 ne réexpriment p53. Toutefois, on a pu mettre en évidence le fait que les cellules US3 et US4 montraient l'activation de WAF-1 par rapport aux cellules parentales malignes U937. Une telle activation de WAF-1 dans une voie indépendante de p53 alternative a été récemment décrite et les résultats actuels montrent que les clones US3 et US4 utilisent, semble-t-il, cette voie alternative WAF-1.

Le gène sina est activé par le type sauvage p53 inductible dans les cellules M1 de même que dans les cellules KS qui réexpriment le type sauvage p53.

Tandis que les cellules parentales U937 eapriment très légèrement l'ARNm de sina, il est activé dans les clones filles US3 et US4 qui ont une réversion de leur phénotype malin et qui réexpriment p21^{waf-1}.

De façon intéressante, sina est activé dans les cellules qui deviennent apoptotiques, comme cela est montré par un double marquage utilisant une sonde sina pour hybridation in situ combinée avec un essai TUNEL

Ceci permet de démontrer que le gène sina humain qui est très conservé dans la phylogénic joue un rôle dans l'apoptose et la suppression tumorale.

De façon encore plus importante, sina se situe au croisement des voies de p53 et de WAF-1.

En outre, en utilisant le modèle de U937 et US3 et US4, on a pu montrer un lien fonctionnel pour les molécules suppresseurs en utilisant un modèle biologique global qui permet la comparaison à des niveaux moléculaires entre les cellules malignes parentales et les cellules filles directement dérivées. Ces expériences indiquent qu'il n'est pas nécessaire de transférer les gènes suppresseurs de tumeur humains spécifiques de façon à eur conférer le phénotype suppresseur, mais que la réversion tumorale est sous le contrôle d'un système de régulation qui est toujours présent dans le matériel génétique des cellules tumorales bien qu'il soit nécessaire de le réactiver.

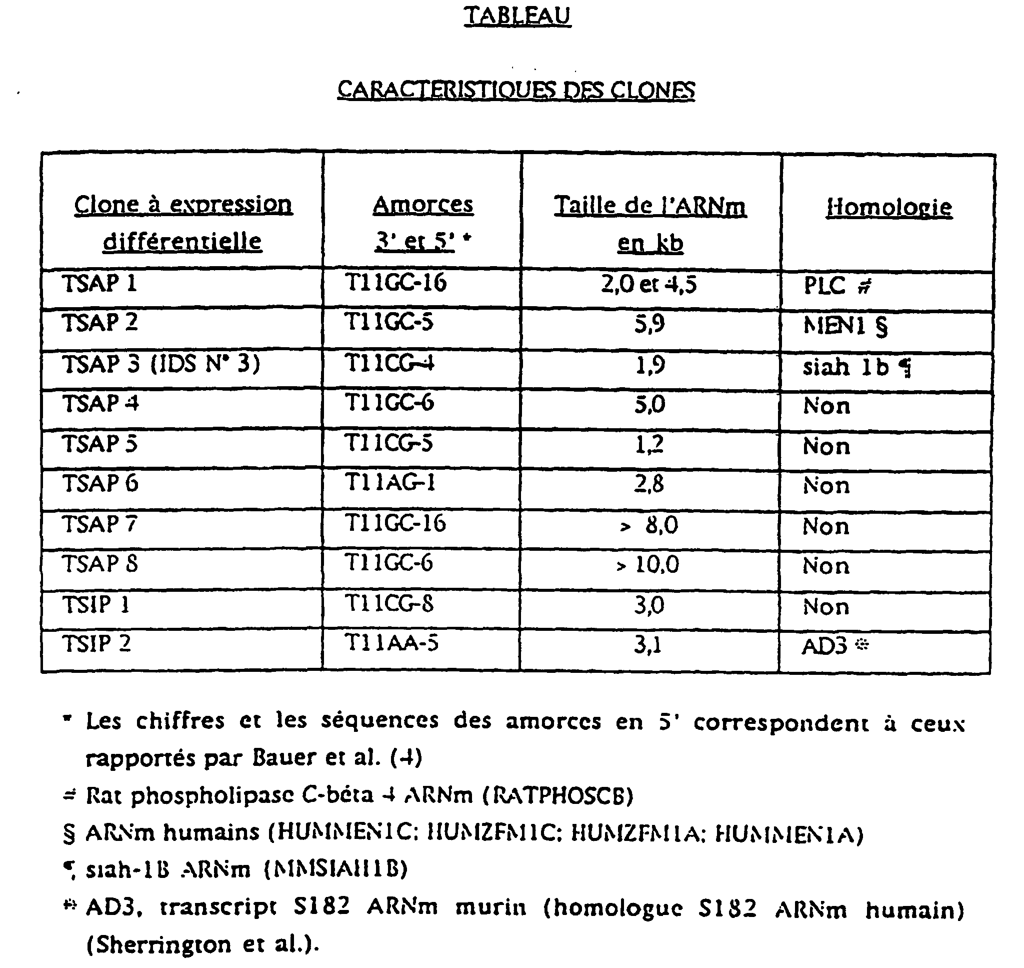

INFORMATIONS POUR LA SEQ ID N° : 1
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSAP 1
      (B) EMPLACEMENT:
   (xi) DESCRIPTION DE LA SEQUENCE: SEQ ID N° 1 : 
TSAP1

10.                                                    TGATCACGTAC

                                                          :  ::::

ratPLC    CTTCTTCTACTTAACAATTTGACTATTGAATTTCTTTGGCCAACCAAAAGTAGCTATGTAC

           3970      3980      3990      4000      4010      4020

           20        30        40        50        60        70

TSAP1     ACACACACACACAGAGAGAGAGAGAGAGAGAGAGGGGAGAGAGAGAGAGAGAGAT

          :.:::::::::::: : : : : : : :            : : : : : : : :.: ::

ratPLC    ACACACACACACACACACACACACACA------------CACACACACACACACAGAAAT

           4030      4040                  4050      4060

           80        90        100       110       120       130

TSAP1     CCCCTATTCCTGACAGGCAGAGTTGAATCATGATATATGGCTTAAACATGTTTGCTATGA

          :::::::::::::::::::::::::::: ::: ::  :   ::::::::::: :::. :

ratPLC    CCCCTATTCCTGACAGGCAGAGTTGAACCATAATCCACAACTTAAACATGTTGGCTAGGG

     4070      4080      4090      4100      4110      4120
 
140 150 160 170 180 190

TSAP 1 GACAGCATCACAAGCCAGTGGGCTTGGTGATAACAACTCTGCTTTGTGGTGCATTAGGAC

::::::::::::::::::::::::::::::::::::::::::::::::::::::::::::

ratPLC GACAGCATCACAAGCCAGTGGGCTTGGTGATAACAACTCTGCTTTGTGGTGCATTAGGAC

4130 4140 4150 4160 4170 4180

200 210 220 230

TSAP1 1 ATTTTTGAGCTGCTGCTGCTGCAAA-AAAAATAAGAGCCG

:: :: ::::::::::: : ::: ::::: :: ::

ratPLC ATGTTCGAGCTGCTGCTG--GAAAAGGAAAATTAGTGCATTAGTACTTTAATGGCAAGCG

4190 4200 4210 4220 4230 4240
INFORMATIONS POUR LA SEQ ID N° : 2
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSAP 2
      (B) EMPLACEMENT:
   (xi) DESCRIPTION DE LA SEQUENCE: SEQ ID N° 2 : 
TSAP2

10          20          30          40          50          60

TSAP2       GCTTGGAACCAATCTACAACAGCGAGGGGAAGCGGCTTAACACTCGAGAGTTCCGTACCC

                 :: :: :::::::: ::::::::::::::::::::::: ::::::::::: ::::

humzfmlc.seq CCCCTGAGCCCATCTACAATAGCGAGGGGAAGCGGCTTAACACCCGAGAGTTCCGCACCC

            250       260       270       280       290       300
 
70 80 90 100 110 120

TSAP2 GCAAAAAAAAAAATCTCTTGTGTTTTCCTAAGCTTTTCCCTGTGCTAGGGAAAGATCAGT

:::::::

humzfmlc.seq GCAAAAAGCTGGAAGAGGAGCGGCACAACCTCATCACAGAGATGGTTGCACTCAATCCGG

310 320 330 340 350 360

130 140

TSAP2 AAGTCCGTGGTTATAGATTGGTT

humzfmlc.seq ATTTCAAGCCACCTGCAGATTACAAACCTCCAGCAACACGTGTGAGTGAT

370 380 390 400 410
INFORMATIONS POUR LA SEQ ID N° : 3
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSAP 3
      (B) EMPLACEMENT:
   (xi) DESCRIPTION DE LA SEQUENCE: SEQ ID N° 3 : 
TSAP3

10          .          .          .          .          .

TSAP3 3                                                                                    TTT[?]TTTTTTTG

                                                                                                  ::::

mmsiah1b.seq TTGTAAAATATTTCTGAACTTTGTATTTGTTGTAGATTGATTGTATTGTTGACAATTTTT

                1450      1460      1470      1480      1490      1500
 
20          30          40          50          60          70

TSAP  3          CGGGGTGGGGGTGTGCCTGCACACATGCGTGCACGTGTGTGCTTGGTTTTCCTTTAACAA

                 ::::::::::::::::::::::::::::::::::::::::::::::::::::::::::::

mmsiah1b.seq  CGGGGTGGGGGTGTGCCTGCACACATGCGTGCACGTGTGTGCTTGGTTTTCCTTTAACAA

              1510        1520        1530        1540        1550        1560

              80          90          100         110         120         130

TSAP  3          GCCATCTACGTGTCATAGCCCACTGTTTTCCCCTTGTGAGTCAACACATAGTGCTGCTGT

                 ::::::::::::::::::::::::::::::::::::::::::::::::::::::::::::

mmsiah1b.seq  GCCATCTACGTGTCATAGCCCACTGTTTTCCCCTTGTGAGTCAACACATAGTGCTGCTGT

              1570        1580        1590        1600        1610        1620

             140

TSAP3         GGTTTGGGTTTGGT

              ::::: ::::::::

mmsiah1b.seq  GGTTTTGGTTTGGTTTGCTTTTGGTTTTGATGTGTGTGTATTTGATAATTTTTATTCTA

              1630        1640        1650        1660        1670        1680
INFORMATIONS POUR LA SEQ ID N° : 4
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSAP 4
      (B) EMPLACEMENT:
   (xi) DESCRIPTION DE LA SEQUENCE: SEQ ID N° 4 : 
TSAP4

AACTCCGTCG TGGGTGTGGG GACCTAATTC CTTATATTTT TACAACAAGC ACTGTACAAA 60

CTGTGCCTTT CCCTAATGCA GTTATACTAT TTCCATTAAG ATGGGTAACC TTAGTTAAGG 120

CTTTATATTC ACTGCCATGG GTAGGAATGC TCACGGTGAA TGGGCCAACT TGTCATGGAA 180

GAAGCCCTCA TTTTCAGTTG GC 202
INFORMATIONS POUR LA SEQ ID N°: 5
   (i) CARACTERISTIQUES DE LA SEQUENCE:
      (A) LONGUEUR:
      (B) TYPE: nucléotide
      (C) NOMBRE DE BRINS: simple
      (D) CONFIGURATION: linéaire
   (ii) TYPE DE MOLECULE : ADNc
   (ix) CARACTERISTIQUE:
      (A) NOM/CLE: TSAP 5
      (B) EMPLACEMENT:
   (xi) DESCRIPTION DE LA SEQUENCE: SEQ ID N° 5 : 
TSAP5

TAACAAGGAT ATTCAGGTTC GGGATTGGTT TCCTAAGCGA TGATCTCAAC CTCCACGTGG 60

AACTGATTTC CCAAGGGACA GAAATGGTCT TTGATCTTTC TGAACCACTT GTCTTCAAAC 120

TCTTTGGAGG ACGCAACCAC CATGGCAGTC AGGGCTCCGG GGCCCACACA CTTCACCTCC 180

GAATGAAGCT CCTCTTTTAT CTTTTCTGGG ACAATGTCTT CCCCCATAGC CTCCTCCATC 240

AACAGCAAAG TACCTTCCCT AAAGTTGAAG TCCTTCACTT TCCCTGCAAT TTCCTGCTGA 300

GTCCTCAAGT TCTTCTCCAA CGCGAATGAT GTTTGCTGAG ACTGGGCGAG CTGAAGCAGG 360

AGCCTGGCGC GGAGCAAAAA GGCGCATGCT TTCCTCCGAG CCTCCATCTG TGCCTCTTCC 420

CTCCGCCTTG CCAGGGAAGG CATATTCTC CTGAGCACTA CCACTCGCTT CCACGGAGAG 480

CAGTGCATTC TCAGGCAAGG TCGTGGGCAA AGACAAAAGA GAGCCTGTTC CCGAGTGTAC 540

AGAGGAGGGA CCGACGGCCT TGTCACTTGA GGCAGAACTC TTCTGTCCCT GCGGTGACAC 600

CCTGCTGGCA GGCCGGGCCC TGGACTCAGG TATGCCTCTG CCAGCTTACA CCAGCTCCAC 660

GGGTTGAGCG GGTGCAAAGC AATCAGCTTG TGCAGGCAGA AGATCGTGTG CTCCCGGCTC 720

TGCAGGCTGG AAAAGACGGC CAGGTGGAGG TGGAGCACCA CGGTCAGATG GTCTGTGTTG 780

GTGGCTTTGC TTTCCAAGTC TGCCGCCATC TCCAGCGCCT CCTCATGCCT CCCAAGTGAG 840

CCAGACACCG AGCCTGGCCT TCTTGGACAT CCCTTTTCAT GGCAAAATTA GTAGATGGTA 900

ATGTTCGGAG ATATGGAGTA TTCCTGCAGG GCTTTCTCGT ATTCCTGTCG TCTGTAGGCC 960

AGGTCCCCTC TGAATTTCTT GAGAGTGAGA ACTTCAATAT CGTCACTACA TTCTGTCTCT 1020

TCATAAAACC ATGCGGCTCG CAGAGCTTGG CGCGGTAGGG GGAGGGCGGC TCGGGCCGGC 1080

GCTCCGGCCT CTGCTCGAAC ACCGAGTCCT CAAATTCGCC GCCCAGCACC CAGCATCCGG 1140

TCTCCATCGC GCGGAAGTGC AACTGGACCT CGAAACGAGG CGACACCTAG AGCGACGCCC 1200

ATCACCCAGC CTCCAAAGCG CGCGACAGCA GCCGCGCCAA GGCTGCCGAG GCAAGGTAGA 1260

GACCTGCCCG GGCGGCCGCT CGAGCCCTAT AGTGAGTCGT ATTAGGATGG 1310
INFORMATIONS POUR LA SEQ ID N° : 6
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSAP 6
      (B) EMPLACEMENT:
   (xi) DESCRIPTION DE LA SEQUENCE: SEQ ID N° 6 : 
TSAP6

GTGAGTACAT ATCACATGTA TGGGGTGTCA TTCTGAGTAT GTCAGTTTAC ACCTGCATCC 60
CAGGAATTAG GATCTCAGCC ACCCACGCAT ATATCATCAC CTCGCTGTGC AGCATCCAGA 120
AAAGAGACCC GAACCCAGCT CAGGGCCCCC ACAAGCCATC TCCACTTCCA GGGCCTCACA 180
CGTGGCTTGT TTTCTCCCCC TGTGTGTGGT CGCCGGACAG CATGAACTTG ACAGCCCCAT 240
CTTTCTCCCA GCCCCTGCGG ATCTTGGTGA GTCTGCGGTT TGAGGCAGGG CAGGAGGAAG 300
AGGCCCTTGG CCAGGATGAT TCACACAGGG GCAGGGAGCA GCGTGAGTGT GGAATGTGGG 360
GCGGGCAGGT AGAACTTGKT AGTGGTTTTT CCTNCAAAAG GCACGGGTCC AGCCGTAGGT 420
GAGTGTGTGC ATTGTGCTGA GTATCAGGGC CACGAAGCCC AGTGTGGACT GCACGAAGCT 480
GAACTCCTTC CAGTTGAGGG AATTAGCAAT GGACGGGAGC GAGGTGACAG CCAGCAGCGA 540
CAACATGCCC AGGGCCAGCA CACCCAGGGA CAGGTATATC TCCATCCTCC AGACTTCTTC 600
CTCAGCCCAG AGGCGGCTCT TGTTGGCCAG GACCTGCTTC ACAGCCAGAT TGACCAGGTC 660
GTAGGCGGTG GGAGCGGCGC AGCGGCAGGC AGAAGCTGTA GAGAGCGTGC AGCATCGGGA 720
AGAAGAAGCT GAGCAGCCCG ATCTGCTTGC GATGCTGCAG CCAGTGGTCC AGCCAGTCTG 780
GGAAGCGCTG GTACTTGGTC CCCCTCCGCA GCTGAAGCGC AGCTGCCAGC ACACCGGGCA 840
GGTACACTAG GGACAGCAGC ACATAAGCCA CACAGGGTAG TGTGGTGTTG ACCACAGACA 900
AGGGCATCTT GTAAAACTTG TTCTCATCTT TCCGAATGTN TGGCTGTANA ACGTGCGGGA 960
TGAAATTGTA GGTGTANAAN CACACAAAGA CCCCAGTGCC CAGGAAGGTG GGCCCCTTCC 1020
 
AGAATGGAAG GAAGCNCAGG GGTTTNGCTT CTACCTCCCT CNCTGAAGCC CANGGATCCA 1080

TNTCCAGGGG TTNAAACCAT NGGGCGTGCA TCTCTGAAAA TGGTCNCTTG GNTTCTGGTN 1140

GATCANTGCA AATAACNCCT GCCTGTTCCN TCCCTTGGGG CCACCCTNTN GGGGCCATGC 1200

CAA 1203
INFORMATIONS POUR LA SEQ ID N°: 7
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSAP 7
      (B) EMPLACEMENT:
   (xi) DESCRIPTION DE LA SEQUENCE: SEQ ID N° 7 : 
TSAP7

GCCCATCCAG TCATTCTTTA TTTCAGTGTG TGAAAGCCTC CTACGCATTT TCCCCCAAAT 50

TAATTTTTAA TCCATTTTCA AACCAGCCTT TACTGTGGCC TTTTCTGCTA TTTTTGATAT 120

ATGTTAGCAC GTGTGCATAG 140
INFORMATIONS POUR LA SEQ ID N° : 8
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSAP 8
      (B) EMPLACEMENT:
   (xi) DESCRIPTION DE LA SEQUENCE: SEQ ID N° 8 : 
TSAP8

CACGTNAAAG TACCACATCC NCCCCCATTG GTAGATATTG ANAGAGTATA TANATAGGNC 60

GAAGCACAAT CTCTTCCCTT CCTNTGTACA CCTCANACCC AGTGACTTCC NACCNAAGCN 120

CNTGANTGTN TTTGTNGATA TGAGTGTCTG NGTGTGTGNA TNTGCGTCTC ACATGTATGG 180

GACGACCNAC CCCACCCCCA GCGGCCTTCA NGCACAATNG AGGACGCCTA TNGTGGATAC 240

GNGCATCGGT AAANAGC 257
INFORMATIONS POUR LA SEQ ID N° : 9
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSIP 1
      (B) EMPLACEMENT:
   (xi) DESCRIPTION DE LA SEQUENCE: SEQ ID N° 9 : 
TSIP1

GGAGGGGGTC TAGCTTTCTC TTTAGTTATC ACTCTGAGGT GCTCAGGTCA CAGAGAAGGC 60

ACTTAATTGG GAAGGTCATC TGATTCCGGC CATCTTCTCT CCCTTTACCA A 111
INFORMATIONS POUR LA SEQ ID N° : 10
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE: ADNc
   (ix) CARACTERISTIQUE:
      (A) NOM/CLE: TSIP 2
      (B) EMPLACEMENT:
   (xi) DESCRIPTION DE LA SEQUENCE: SEQ ID N° 10 : 
TSIP2

CACCGGTGAGACCTCTAGGGCGGGGCCTAGGACGACCTGCTCCGTGGGCCGCGAGTATTC 60
GTCGGAAACAAAACAGCGGCAGCTGAGGCGGAAACCTAGGCTGCGAGCCGGCCGCCCGGG 120
CGCGGAGAGAGAAGGAACCAACACAAGACAGCAGCCCTTCGAGGTCTTTAGGCAGCTTGG 180
AGGAGAACACATGAGAGAAAGAATCCCAAGAGGTTTTGTTTTCTTTGAGAAGGTATTTCT 240
GTCCAGCTGCTCCAATGACAGAGATACCTGCACCTTTGTCCTACTTCCAGAATGCCCAGA 300
TGTCTGAGGACAGCCACTCCAGCAGCGCCATCCGGAGCCAGAATGACAGCCAAGAACGGC 360
AGCAGCAGCATGACAGGCAGAGACTTGACAACCCTGAGCCAATATCTAATGGGCGGCCCC 420
AGAGTAACTCAAGACAGGTGGTGGAACAAGATGAGGAGGAAGACGAAGAGCTGACATTGA 480
AATATGGAGCCAAGCATGTCATCATGCTCTTTGTCCCCGTGACCCTCTGCATGGTCGTCG 540
TCGTGGCCACCATCAAATCAGTCAGCTTCTATACCCGGAAGGACGGTCAGCTAATCTACA 600
CCCCATTCACAGAAGACACTGAGACTGTAGGCCAAAGAGCCCTGCACTCGATCCTGAATG 660
CGGCCATCATGATCAGTGTCATTGTCATTATGACCATCCTCCTGGTGGTCCTGTATAAAT 720
ACAGGTGCTACAAGGTCATCCACGCCTGGCTTATTATTTCATCTCTGTTGTTGCTGTTCT 780
TTTTTTCGTTCATTTACTTAGGGGAAGTATTTAAGACCTACAATGTCGCCGTGGACTACG 840
TTACAGTAGCACTCCTAATCTGGAATTTTGGTGTGGTCGGGATGATTGCCATCCACTGGA 900
AAGGCCCCCTTCGACTGCAGCAGGCGTATCTCATTATGATCAGTGCCCTCATGGCCCTGG 960
TATTTATCAAGTACCTCCCCGAATGGACCGCATGGCTCATCTTGGCTGTGATTTCAGTAT 1020
ATGATTTGGTGGCTGTTTTATGTCCCAAAGGCCCACTTCGTATGCTGGTTGAAACAGCTC 1080
AGGAAAGAAATGAGACTCTCTTTCCAGCTCTTATCTATTCCTCAACAATGGTGTGGTTGG 1140
TGAATATGGCTGAAGGAGACCCAGAAGCCCAAAGGAGGGTACCCAAGAACCCCAAGTATA 1200
ACACACAAAGAGCGGAGAGAGAGACACAGGACAGTGGTTCTGGGAACGATGATGGTGGCT 1260
TCAGTGAGGAGTGGGAGGCCCAAAGAGACAGTCACCTGGGGCCTCATCGCTCCACTCCCG 1320
AGTCAAGAGCTGCTGTCCAGGAACTTTCTGGGAGCATTCTAACGAGTGAAGACCCGGAGG 1380
AAAGAGGAGTAAAACTTGGACTGGGAGATTTCATTTTCTACAGTGTTCTGGTTGGTAAGG 1440
CCTCAGCAACCGCCAGTGGAGACTGGAACACAACCATAGCCTGCTTTGTAGCCATACTGA 1500
TCGGCCTGTGCCTTACATTACTCCTGCTCGCCATTTTCAAGAAAGCGTTGCCAGCCCTCC 1560
CCATCTCCATCACCTTCGGGCTCGTGTTCTACTTCGCCACGGATTACCTTGTGCAGCCCT 1620
TCATGGACCAACTTGCATTCCATCAGTTTTATATCTAGCCTTTCTGCAGTTAGAACATGG 1680
ATGTTTCTTCTTTGATTATCAAAAACACAAAAACAGAGAGCAAGCCCGAGGAGGAGACTG 1740
GTGACTTTCCTGTGTCCTCAGCTAACAAAGGCAGGACTCCAGCTGGACTTCTGCAGCTTC 1800
CTTCCGAGTCTCCCTAGCCACCCGCACTACTGGACTGTGGAAGGAAGCGTCTACAGAGGA 1860
INFORMATIONS POUR LA SEQ ID N° : 11
   (i) CARACTERISTIQUES DE LA SEQUENCE:
      (A) LONGUEUR:
      (B) TYPE: nucléotide
      (C) NOMBRE DE BRINS: simple
      (D) CONFIGURATION : linéaire
   (ii) TYPE DE MOLECULE : ADNc
   (ix) CARACTERISTIQUE:
      (A) NOM/CLE: TSAP 3 humain
      (B) EMPLACEMENT:
   (xi) DESCRIPTION DE LA SEQUENCE: SEQ ID N° 11 :

TSAP3 humain
m s r q t a t a l p t g t s k c p p s q
atgagccgtcagactgctacagcattacctaccggtacctcgaagtgtccaccatcccag
10 20 30 40 50 60

r v p a l t g t t a s n n d l a s l f e
agggtgcctgccctgactggcacaactgcatccaacaatgacttggcgagtctttttgag
70 80 90 100 110 120

c p v c f d y v l p p i l q c q s g h l
tgtccagtctgctttgactatgtgttaccgcccattcttcaatgtcagagtggccatctt
130 140 150 160 170 180

v c s n c r p k l t c c p t c r g p l g
gtttgtagcaactgtcgcccaaagctcacatgttgtccaacttgccggggccctttggga
190 200 210 220 230 240

s i r n l a m e k v a n s v l f p c k y
tccattcgcaacttggctatggagaaagtggctaattcagtactttttccctgtaaatat
250 260 270 280 290 300

a s s g c e i t l p h t e k a d h e e l
gcgtcttctggatgtgaaataactctgccacacacagaaaaagcagaccatgaagagctc
310 320 330 340 350 360

c e f r p y s c p c p g a s c k w q g s
tgtgagtttaggccttattcctgtccgtgccctggtgcttcctgtaaatggcaaggctct
370 380 390 400 410 420

l d a v m p h l m h q h k s i t t l q g
ctggatgctgtaatgccccatctgatgcatcagcataagtccattacaaccctacaggga
430 440 450 460 470 480

e d i v f l a t d i n l p g a v d w v m
gaggatatagtttttcttgctacagacattaatcttcctggtgctgttgactgggtgatg
490 500 510 520 530 540

m q s c f g f h f m l v l e k q e k y d
atgcagtcctgttttggctttcacttcatgttagtcttagagaaacaggaaaaatacgat
550 560 . 570 580 590 600

g h q q f f a i v q l i g t r k q a e n
ggtcaccagcagtttttcgcaatcgtacagctgataggaacacgcaagcaagctgaaaat
610 .620 630 640 650 660

f a y r l e l n g h r r r l t w e a t p
tttgcttaccgacttgagctaaatggtcataggcgacgattgacttgggaagcgactcct
670 680 690 700 710 720

r s i h e g i a t a i m n s d c l v f d
cgatctattcatgaaggaattgcaacagccattatgaatagcgactgtctagtctttgac
730 740 750 760 770 780

p a l h s f l q t n g n l g i n v t i s
ccagcattgcacagcttttgcagacaaatggcaatttaggcatcaatgtaactatttcc
790 800 810 820 830 840

m c *
atgtgttgaaatggcaatcaaacatttctggccagtgtttaaaacttcagtttcacaga
850 860 870 880 890 900

aaataaggcacccatctgtctgccaacctaaaactctttcggtaggtagaagctcgacat
910 920 930 940 950 960

gaaggccaataaaaagaaagactgctaaatacaggaaacagttccatgtagtaacactaa
970 980 990 1000 1010 1020

tatatttaaaaataagtcaacagtaaaccactgaaaaatatacgtatatacacccaaga
1030 1040 1050 1060 1070 1080

tgggcatcttttgtattaagaaaggaagcattgtaaaataattctgagtttcgtgtttgt
1090 1100 1110 1120 1130 1140

tgtagattgattgtattgttgaaaaagtttgttttgcgtgggagtgtgtgcctgcgtgg
1150 1160 1170 1180 1190 1200

gtgtgtgcgtgtttgggtttttttcctttaactgacaagccatcttgagtggtcatgggc
1210 1220 1230 1240 1250 1260

cattgttttcccctttgtgagtcaatacatagtgctgctgtaagccgttttgtgtgtat
1270 1280 1290 1300 1310 1320

ttgctaatttttattaatttagttttcattaaataaatttgactttctgtaatttag
1330 1340 1350 1360 1370 1380

gtttttcctttttgtaccatttaaagttagtatcttttgatatggcatatttgttta
1390 1400 1410 1420 1430 1440

tggtaaaaaatttataacgggttcaatatttcctttcccccattaatcaagtccattgg
1450 1460 1470 1480 1490 1500

aaatatttaaaaccagcctatttggtgaacccatgagttcccagaaagtaaaggtgac
1510 1520 1530 1540 1550 1560

acccggaaaaataatccaaaagcctatttaaagccacctataaggtgcccccctttcctg
1570 1580 1590 1600 1610 1620

tctttcctacagatgagtcacacctttgagccttaacctttgaaaggttagagaataaatt
1630 1640 1650 1660 1670 1680

gatttttataaatactgcaaatccaggcttttgtttcctttttccagatatccttggaca
1690 1700 1710 1720 1730 1740

aatcatatattttaaatttgttcttgtatttattggttttgcagaagaaggcatcgtca
1750 1760 1770 1780 1790 1800

tgcacagtatttgtaattaaaagcaaattcatttgtttaaaaaggcagtttgcaaaaaat
1810 1820 1830 1840 1850 1860

gtttttggtcttttataattcttca
1870 1880

### REFERENCES BIBLIOGRAPHIQUES

(1) Liang P. & Pardee A.B. (1992) Science, 257, 967-971.
(2) Don R.H., Cox P.T., Wainwright B.J., Baker K. & Mattick J.S. (1991) Nucl. Acids Res., 19, 4008.
(3) Yonish-Rouach E., Resnitzky D., Lotem J., Sachs L, Kimchi A. & Oren M. (1991) Nature 352, 345-347.
(4) Bauer D., Muller H., Reich J., Riedel H., Ahrenkiel V., Warthoe P. & Strauss M. (1993) Nucl. Acids Res. 21, 4272-4280.
(5) Sambrook J., Fritsch E.F. & Maniatis T. (1989) Molecular Cloning: a laboratory manual.
(6) Okamoto K. & Beach D. (1994) EMBO J., 13, 4816-4822.
(7) Angerer L & Angerer R.C. (1991) Methods in cell biology : functional organization of the nucleus, 35, 37-71.
(8) Linares-Cruz G., Rigaut J.P., Vassy J., De Oliveira T.C., De Cremoux P., Olofsson B. & Calvo F. (1994) J. Microsc., 173, 27-38.
(9) Bieche I. and Lidereau R., Genes Chromosomes and Cancer 14, 227-251 (1995).
(10) Wang-Wuu S., Soukup S., Bove K., Gotwals B. and Lampkin B., Cancer Research 50, 2786-2793 (1990).
(11) Maw M.A. et al., Cancer Research 52, 3094-3098 (1992).
(12) Austruy E et al., Cenes, Chromosomes and Cancer, 14, 285-294 (1995).
(13) Kuytek-Black A.E. et al., Nat. Genet 5(4)n 392-396 (1993).
(14) Newsham I. et al., Genes Chromosomes and Cancer 12(1), 1-7, (1995).
(15) Sherrington et al., Nature, vol. 375, p. 754-760 (1995).

## Revendications

1. Utilisation d'un composé capable d'inhiber l'expression de SEQ ID N° 10 choisi dans le groupe comprenant une séquence anti-sens de SEQ ID N° 10 et un anticorps monoclonal dirigé contre la protéine codée par SEQ ID N° 10 pour la préparation d'un médicament destiné au traitement des cancers.

2. Utilisation de cellules transformées par un vecteur d'expression cellulaire de SEQ ID N° 10 pour le criblage de médicaments anti-cancéreux.

3. Utilisation selon la revendication 2, **caractérisée en ce que** le vecteur est un vecteur viral.

4. Utilisation selon la revendication 3, **caractérisée en ce que** le vecteur viral est issu d'un adénovirus, d'un rétrovirus, d'un virus herpès ou d'un poxvirus.

5. Utilisation selon la revendication 2, **caractérisée en ce que** le vecteur est un vecteur à acide nucléique nu.

6. Utilisation in vitro de tout ou partie de SEQ ID N° 10 comme sonde nucléotidique ou comme amorce d'amplification pour la détermination de la prédisposition et le suivi de cancers.

7. Utilisation in vitro d'anticorps dirigé contre la protéine codée par SEQ ID N° 10 pour la détermination de la prédisposition et le suivi de cancers.

## Patentansprüche

1. Verwendung einer Verbindung, die in der Lage ist, die Expression von SEQ ID Nr. 10 zu hemmen, ausgewählt in der Gruppe, umfassend eine Antisinn-Sequenz von SEQ ID Nr. 10 und einen gegen das durch SEQ ID Nr. 10 kodierte Protein gerichteten monoklonalen Antikörper, für die Herstellung eines Arzneimittels, welches für die Behandlung von Krebserkrankungen bestimmt ist.

2. Verwendung von Zellen, die durch einen Vektor für eine zelluläre Expression von SEQ ID Nr. 10 transformiert sind, für das Screening von antineoplastischen Arzneimitteln.

3. Verwendung nach Anspruch 2, **dadurch gekennzeichnet, dass** der Vektor ein viraler Vektor ist.

4. Verwendung nach Anspruch 3, **dadurch gekennzeichnet, dass** der virale Vektor von einem Adenovirus, einem Retrovirus, einem Herpes-Virus oder einem Poxvirus stammt.

5. Verwendung nach Anspruch 2, **dadurch gekennzeichnet, dass** der Vektor ein Vektor mit nackter Nukleinsäure ist.

6. In vitro-Verwendung der Gesamtheit oder eines Teils von SEQ ID Nr. 10 als Nukleotidsonde oder als Amplifizierungsprimer für die Bestimmung der Veranlagung für und die Verfolgung von Krebserkrankungen.

7. In vitro-Verwendung eines gegen das durch SEQ ID Nr. 10 kodierte Protein gerichteten Antikörpers für die Bestimmung der Veranlagung für und die Verfolgung von Krebserkrankungen.

## Claims

1. Use of a compound capable of inhibiting the expression of SEQ ID No: 10, chosen from the group comprising a sequence which is antisense to SEQ ID No: 10 and a monoclonal antibody which is directed against the protein which is encoded by SEQ ID No: 10, for preparing a medicament intended for the treatment of cancers.

2. Use of cells which are transformed by a cellular expression vector of SEQ ID No: 10, for screening for anticancer medicaments.

3. Use according to Claim 2, **characterized in that** the vector is a viral vector.

4. Use according to Claim 3, **characterized in that** the viral vector is derived from an adenovirus, from a retrovirus, from a herpesvirus or from a poxvirus.

5. Use according to Claim 2, **characterized in that** the vector is a naked nucleic acid vector.

6. In vitro use of all or part of SEQ ID No: 10 as nucleotide probe or as amplification primer for determining predisposition to, and for following up, cancers.

7. In vitro use of an antibody which is directed against the protein which is encoded by SEQ ID No: 10, for determining predisposition to, and for following up, cancers.
